# EUROPEAN PATENT SPECIFICATION

(11) **EP 4 426 865 B1**
(45) Date of publication and mention of the grant of the patent: **01.04.2026**
(21) Application number: 22817538.6
(22) Date of filing: 03.11.2022
(51) Int. Cl.: C12Q 1/6893

(54) **COMPOSITIONS AND METHODS FOR DETECTION OF MALARIA**
ZUSAMMENSETZUNGEN UND VERFAHREN ZUM NACHWEIS VON MALARIA
COMPOSITIONS ET PROCÉDÉS POUR LA DÉTECTION DU PALUDISME

(30) Priority: 05.11.2021 US 202163263594 P
(43) Date of publication of application: 11.09.2024
(73) Proprietor: F. Hoffmann-La Roche AG, 4070 Basel (CH)
(72) Inventor: CHIU, Sharon Ho-Chen, Pleasanton, California 94588 (US); GICHERU, Yvonne, Pleasanton, California 94588 (US); HEIL, Marintha, Pleasanton, California 94588 (US); LEE, Michael K., Pleasanton, California 94588 (US); MANOHAR, Chitra, Pleasanton, California 94588 (US); ORDINARIO, Ellen, Pleasanton, California 94588 (US); RAJAMANI, Jaya, Pleasanton, California 94588 (US); SUN, Jingtao, Pleasanton, California 94588 (US)
(74) Representative: Hövelmann, Sascha Alexander
(86) International application number: PCT/EP2022/080736
(87) International publication number: WO 2023/079032

(56) References cited:
- WO-A2-2020/132408
- SEILIE ANNETTE M. ET AL: "Beyond Blood Smears: Qualification of Plasmodium 18S rRNA as a Biomarker for Controlled Human Malaria Infections", THE AMERICAN SOCIETY OF TROPICAL MEDICINE AND HYGIENE, vol. 100, no. 6, 5 June 2019 (2019-06-05), US, pages 1466 - 1476, XP093026422, ISSN: 0002-9637, Retrieved from the Internet <URL:https://www.ncbi.nlm.nih.gov/pmc/articles/PMC6553913/pdf/tpmd190094.pdf> DOI: 10.4269/ajtmh.19-0094
- KAMAU EDWIN ET AL: "Development of a Highly Sensitive Genus-Specific Quantitative Reverse Transcriptase Real-Time PCR Assay for Detection and Quantitation of Plasmodium by Amplifying RNA and DNA of the 18S rRNA Genes", JOURNAL OF CLINICAL MICROBIOLOGY, vol. 49, no. 8, 1 August 2011 (2011-08-01), US, pages 2946 - 2953, XP093026440, ISSN: 0095-1137, Retrieved from the Internet <URL:https://journals.asm.org/doi/pdf/10.1128/JCM.00276-11> DOI: 10.1128/JCM.00276-11
- J. J. WAGGONER ET AL: "Multiplex Nucleic Acid Amplification Test for Diagnosis of Dengue Fever, Malaria, and Leptospirosis", JOURNAL OF CLINICAL MICROBIOLOGY, vol. 52, no. 6, 26 March 2014 (2014-03-26), pages 2011 - 2018, XP055199526, ISSN: 0095-1137, DOI: 10.1128/JCM.00341-14

## Description

The instant application contains a Sequence Listing, which has been submitted electronically in XML format. Said XML copy, created on November 1, 2022, is named "P36987-WO sequence listing" and is 65,548 bytes in size.

### FIELD OF THE INVENTION

The present disclosure relates to the field of *in vitro* diagnostics. Within this field, the present invention concerns the amplification and detection of a target nucleic acid that may be present in a sample and particularly, the amplification, detection, and quantitation of a target nucleic acid comprising sequence variations and/or individual mutations of Malaria parasites, using primers and probes. The invention further provides reaction mixtures and kits containing primers and probes for amplification and detection of Malaria.

### BACKGROUND OF THE INVENTION

Malaria is a mosquito-borne infectious disease that affects humans and other animals. Malaria is a disease caused by the single-celled microorganism *Plasmodium* parasite that is transmitted to humans after being bitten by infected female Anopheles mosquitoes. Initial symptoms of Malaria include fever, headache, and chills, and can be treated with available anti-malarial drugs. If left untreated, severe Malaria can develop, with symptoms including anemia, cerebral malaria, and respiratory distress, which can ultimately result in death.

According to the 2018 World Health Organization (WHO) Report, roughly 3.2 billion people are at risk for Malaria, across 87 countries, which are primarily located in tropical and subtropical regions. There are roughly 219 million cases of Malaria per year, with 92% of those cases coming from Africa, 5% in Southeast Asia, and 2% in East Mediterranean countries. The U.S. presents with about 1,700 cases per year. Malaria also results in 435,000 fatalities a year, with 266,000 of those fatalities striking children under five years old. Efforts to eradicate and prevent Malaria are ongoing, which include insecticide-treated nets, increasing patient access to healthcare, drug development vaccines, and genetically-modified mosquitoes (GMO). Although the U.S. is not considered an endemic country for Malaria, global travel to/from the U.S. may lead to an increase in cases of imported Malaria.

There are at least five species of *Plasmodium* that are known to infect humans: *P. falciparum*, *P. vivax*, *P. ovale*, *P. malariae*, and *P. knowlesi.* Clinical presentations can vary, depending on the species of *Plasmodium. P. falciparum* is the most deadly species, which has been known to cause rapid cell division in infected red blood cells, which causes anemia and blocked blood vessels to the brain. The *Plasmodium* life cycle is complex, with the parasite alternating between sexual reproduction in the mosquito and asexual reproduction in the human host. Symptoms are caused by the parasite during the blood stages and can appear between 7-30 days after a mosquito bite. *P*. *vivax* and *P. ovale* can remain dormant in the liver stage, re-activating months or even years later. With respect to blood supply and blood donations, there already exists major challenges to maintain sufficient blood supply with critical shortages in the Southern and Eastern U.S. Malaria presents an additional problem for blood supply and blood donations. For example, while the U.S. is considered a non-endemic country, global travel may lead to an increase in the cases of imported Malaria, thereby affecting the blood supply in the U.S. Worldwide, the current strategies regarding Malaria employed to maintain safety of blood supply include: (1) Selective Testing, and (2) Donor Deferral, with the U.S. following a Donor Deferral policy. Typically, donors are deferred based on responses to the screening questionnaire, based on history, residence, and travel. In the U.S., an estimated 191,000 donors are temporarily deferred (1-3 years) with the majority of deferrals attributed to traveling to endemic areas. Moreover, deferred donations negatively impact the blood supply, because roughly only ¼ of donors who are temporarily deferred actually return for subsequent blood donation after the deferral period has concluded. Donor deferrals negatively impact the blood supply by excluding donations based on questionnaire answers, regardless of Malaria status. On the other hand, selective testing reduces the impact on blood supply inventory as compared to donor deferral. Selective testing of blood donations is also based on questionnaire responses, but the selective testing policy allows for shorter deferral times and reinstatement of donors depending on donor's Malaria status. In countries that have selective testing policy, testing is done by enzyme immunoassays. At least three countries have implemented testing of Malaria in blood donations: France, England, and Australia. In France, of the 3 million donations one year, about 180,000 donations were tested, and of those 180,000 tested donations, about 3,300 (or 1.8%) were positive.

Several diagnostic and screening methods exist for Malaria. Detection of Malaria by microscopy of Giemsa-stained blood smears allows for species identification, is inexpensive, and remains the gold standard. However, microscopy is not capable of high throughput screening/detection. Rapid Diagnostic Tests (RDT) that employ methods for immunochromatographic detection of Malaria antigens are fast and inexpensive, but exhibit low sensitivity (200-5,000 p/µL) and still require confirmation by microscopy. Enzyme Immunoassays (EIA) that detect anti-Plasmodium antibodies in serum are widely used, but exhibit low sensitivity as well. By contrast, nucleic acid tests (NAT) are suitable for high throughput screening/detection and are highly sensitive (< 1 p/µL). Thus, nucleic acid tests represent the best way to detect and screen for blood samples for Malaria, owing to its high sensitivity and high throughput capabilities. However, at present, there are no *in vitro* diagnostic nucleic acid tests available for screening blood donations. Thus, there is a need in the art for a quick, reliable, specific, and sensitive method for detecting and quantifying the presence of Malaria in biological samples, such as blood.

In the field of molecular diagnostics, the amplification and detection of nucleic acids is of considerable significance. Such methods can be employed to detect any number of microorganisms, such as viruses and bacteria. The most prominent and widely-used amplification technique is the Polymerase Chain Reaction (PCR). Other amplification techniques include Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), and Qβ-amplification. Automated systems for PCR-based analysis often make use of a real-time detection of product amplification during the PCR process in the same reaction vessel. Key to such methods is the use of modified oligonucleotides carrying reporter groups or labels.

The absence of a reliable, quick, inexpensive, and sensitive means for detecting Malaria, threatens the safety of the blood donor supply. Therefore, there is a need in the art for a quick, reliable, specific, and sensitive method for detecting and quantifying the presence of Malaria in biological samples, such as blood. This present disclosure is directed to a polymerase chain reaction (PCR)-based assay to detect and screen all *Plasmodium* spp (*P. falciparum, P. vivax, P. ovale, P. knowlesi,* and *P. malariae*) in blood samples and donations.

### SUMMARY OF THE INVENTION

The present invention is defined by the appended claims.

Certain embodiments in the present disclosure relate to methods for the rapid detection of the presence or absence of Malaria parasites (such as *Plasmodium*) in a biological or non-biological sample, for example, multiplex detection and quantitating of Malaria parasites (such as *Plasmodium*) by real-time polymerase chain reaction (PCR) in a single test tube or vessel. Embodiments include methods of detection of Malaria parasites (such as *Plasmodium*) comprising performing at least one cycling step, which may include an amplifying step and a hybridizing step. Furthermore, embodiments include primers, probes, and kits that are designed for the detection of Malaria parasites (such as *Plasmodium*) in a single tube or vessel.

One embodiment is directed to a method for detecting one or more Malaria parasite species in a sample, the method comprising: (a) performing an amplification step comprising contacting the sample with one or more sets of oligonucleotide primers to produce an amplification product, if a target nucleic acid of the one or more Malaria parasite species is present in the sample; (b) performing a hybridization step comprising contacting one or more oligonucleotide probes with the amplification product, if a target nucleic acid of the one or more Malaria parasite species is present in the sample; and (c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and wherein the one or more sets of oligonucleotide primers and the one or more oligonucleotide probes comprise: (1) a set of oligonucleotide primers comprising oligonucleotide primers comprising or consisting of the nucleic acid sequences of SEQ ID NOs:1 and 2, or complements thereof; and an oligonucleotide probe comprising or consisting of the nucleic acid sequence of SEQ ID NO:3, or a complement thereof; and/or (2) a set of oligonucleotide primers comprising oligonucleotide primers comprising or consisting of the nucleic acid sequence of SEQ ID NOs:13 and 14, or complements thereof; and an oligonucleotide probe comprising or consisting of the nucleic acid sequence of SEQ ID NO:15, or a complement thereof. In another embodiment, the one or more Malaria parasite species belongs to the genus *Plasmodium.* In another embodiment, the one or more Malaria parasite species that belong to the genus *Plasmodium* is *P. falciparum, P. vivax, P. ovale, P. malariae,* and/or *P. knowlesi.* In another embodiment, the sample is a biological sample. In another embodiment, the biological sample is whole blood, respiratory specimens, urine, fecal specimens, blood specimens, plasma, dermal swabs, nasal swabs, wound swabs, blood cultures, skin, or soft tissue infections. In another embodiment, the biological sample is whole blood. In another embodiment, at least one of the one or more oligonucleotide probes is labeled. In another embodiment, the at least one of the one or more oligonucleotide probes is labeled with a donor fluorescent moiety and a corresponding acceptor moiety. In another embodiment, step (c) further comprises detecting the presence or absence of fluorescent resonance energy transfer (FRET) between the donor fluorescent moiety and the acceptor moiety of the one or more oligonucleotide probes, wherein the presence or absence of fluorescence is indicative of the presence of absence of the one or more Malaria parasite species in the sample.

Another embodiment is directed to a method for detecting a first target nucleic acid and/or a second target nucleic acid of one or more Malaria parasite species in a sample, the method comprising: (a) performing an amplification step comprising contacting the sample with one or more sets of oligonucleotide primers to produce an amplification product, if the first target nucleic acid and/or the second target nucleic acid of the one or more Malaria parasite species is present in the sample; (b) performing a hybridization step comprising contacting one or more oligonucleotide probes with the amplification product, if the first target nucleic acid and/or the second target nucleic acid of the one or more Malaria parasite species is present in the sample; and (c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and wherein the one or more set of oligonucleotide primers and the one or more oligonucleotide probes comprise: (1) a set of oligonucleotide primers and a oligonucleotide probe for the first target nucleic acid of the one or more Malaria parasite species, wherein the set of oligonucleotide primers comprises oligonucleotide primers comprising or consisting of the nucleic acid sequences of SEQ ID NOs: 1 and 2, or complements thereof; and wherein the oligonucleotide probe comprises or consists of the nucleic acid sequence of SEQ ID NO:3, or a complement thereof; and (2) a set of oligonucleotide primers and an oligonucleotide probe for the second target nucleic acid of the one or more Malaria parasite species, wherein the set of oligonucleotide primers oligonucleotide comprises oligonucleotide primers comprising or consisting of the nucleic acid sequences of SEQ ID NOs: 13 and 14, or a complements thereof; and wherein the oligonucleotide probe comprises or consists of the nucleic acid sequence of SEQ ID NO:15, or a complement thereof. Another embodiment is directed to a method for detecting one or more Malaria parasite species in a sample, the method comprising: (a) performing an amplification step comprising contacting the sample with one or more sets of oligonucleotide primers to produce an amplification product, if a target nucleic acid of the one or more Malaria parasite species is present in the sample; (b) performing a hybridization step comprising contacting one or more oligonucleotide probes with the amplification product, if the target nucleic acid of the one or more Malaria parasite species is present in the sample; and (c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and wherein the one or more sets of oligonucleotide primers and the one or more oligonucleotide probes comprise a set of oligonucleotide primers comprising oligonucleotide primers comprising or consisting of the nucleic acid sequences of SEQ ID NOs:1 and 2, or complements thereof; and an oligonucleotide probe comprising or consisting of the nucleic acid sequence of SEQ ID NO:3, or a complement thereof. Another related embodiment is directed to a method for detecting one or more Malaria parasite species in a sample, the method comprising: (a) performing an amplification step comprising contacting the sample with one or more sets of oligonucleotide primers to produce an amplification product, if a target nucleic acid of the one or more Malaria parasite species is present in the sample; (b) performing a hybridization step comprising contacting one or more oligonucleotide probes with the amplification product, if the target nucleic acid of the one or more Malaria parasite species is present in the sample; and (c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and wherein the one or more sets of oligonucleotide primers and the one or more oligonucleotide probes comprise a set of oligonucleotide primers comprising oligonucleotide primers comprising or consisting of the nucleic acid sequences of SEQ ID NOs: 13 and 14, or complements thereof; and an oligonucleotide probe comprising or consisting of the nucleic acid sequence of SEQ ID NO:15, or a complement thereof. In another embodiment, the one or more Malaria parasite species belongs to the genus *Plasmodium.* In another embodiment, the one or more Malaria parasite species that belongs to the genus *Plasmodium* is *P. falciparum, P. vivax, P. ovale, P. malariae,* and/or *P. knowlesi.* In another embodiment, the sample is a biological sample. In another embodiment, the biological sample is whole blood, respiratory specimens, urine, fecal specimens, blood specimens, plasma, dermal swabs, nasal swabs, wound swabs, blood cultures, skin, or soft tissue infections. In another embodiment, the biological sample is whole blood. In another embodiment, at least one of the one or more oligonucleotide probes is labeled. In another embodiment, the at least one of the one or more oligonucleotide probes is labeled with a donor fluorescent moiety and a corresponding acceptor moiety.

Another embodiment is directed to a kit for detecting one or more Malaria parasite species that may be present in a sample, the kit comprising amplification reagents comprising at least one of a DNA polymerase and nucleotide monomers, and one or more sets of oligonucleotide primers and one or more oligonucleotide probes, wherein the one or more seta of oligonucleotide primers and the one or more oligonucleotide probes comprise: (1) a set of oligonucleotide primers comprising oligonucleotide primers comprising or consisting of the nucleic acid sequences of SEQ ID NOs:1 and 2, or complements thereof; and an oligonucleotide probe comprising or consisting of the nucleic acid sequence of SEQ ID NO:3, or a complement thereof; and/or (2) a set of oligonucleotide primers comprising oligonucleotide primers comprising or consisting of the nucleic acid sequences of SEQ ID NOs: 13 and 14, or complements thereof; and an oligonucleotide probe comprising or consisting of the nucleic acid sequence of SEQ ID NO:15, or a complement thereof. In another embodiment, the one or more sets of oligonucleotide primers and the one or more oligonucleotide probes comprise: (1) a set of oligonucleotide primers comprising oligonucleotide primers comprising or consisting of the nucleic acid sequences of SEQ ID NOs:1 and 2, or complements thereof; and an oligonucleotide probe comprising or consisting of the nucleic acid sequence of SEQ ID NO:3, or a complement thereof; and (2) a set of oligonucleotide primers comprising oligonucleotide primers comprising or consisting of the nucleic acid sequences of SEQ ID NOs: 13 and 14, or complements thereof; and an oligonucleotide probe comprising or consisting of the nucleic acid sequence of SEQ ID NO:15, or a complement thereof. In another embodiment, the one or more Malaria parasite species belongs to the genus *Plasmodium.* In another embodiment, the one or more Malaria parasite species that belongs to the genus *Plasmodium* is *P. falciparum, P. vivax, P. ovale, P. malariae*, and/or *P. knowlesi.* In another embodiment, the sample is a biological sample. In another embodiment, the biological sample is whole blood, respiratory specimens, urine, fecal specimens, blood specimens, plasma, dermal swabs, nasal swabs, wound swabs, blood cultures, skin, or soft tissue infections. In another embodiment, the biological sample is whole blood. In another embodiment, at least one of the one or more oligonucleotide probes is labeled. In another embodiment, the at least one or more oligonucleotide probes is labeled with a donor fluorescent moiety and a corresponding acceptor moiety.

Another embodiment is directed to a kit for detecting a first target nucleic acid and/or a second target nucleic acid of one or more Malaria parasite species that may be present in a sample, the kit comprising amplification reagents comprising at least one of a DNA polymerase and nucleotide monomers, and one or more sets of oligonucleotide primers and one or more oligonucleotide probes, wherein the one or more sets of oligonucleotide primers and the one or more oligonucleotide probes comprise: (1) a set of oligonucleotide primers and a oligonucleotide probe for the first target nucleic acid of the one or more Malaria parasite species, wherein the set of oligonucleotide primers comprises oligonucleotide primers comprising or consisting of the nucleic acid sequences of SEQ ID NOs:1 and 2, or complements thereof; and wherein the oligonucleotide probe comprises or consists of the nucleic acid sequence of SEQ ID NO:3, or a complement thereof; and (2) a set of oligonucleotide primers and an oligonucleotide probe for the second target nucleic acid of the one or more Malaria parasite species, wherein the set of oligonucleotide primers comprises oligonucleotide primers comprising or consisting of the nucleic acid sequences of SEQ ID NOs: 13 and 14, or complements thereof; and wherein the oligonucleotide probe comprises the nucleic acid sequence of SEQ ID NO:15, or a complement thereof. Another embodiment is directed to a kit for detecting one or more Malaria parasite species that may be present in a sample, the kit comprising amplification reagents comprising at least one of a DNA polymerase and nucleotide monomers, and one or more sets of oligonucleotide primers and one or more oligonucleotide probes, wherein the one or more sets of oligonucleotide primers and the one or more oligonucleotide probes comprise: a set of oligonucleotide primers comprising oligonucleotide primers comprising or consisting of the nucleic acid sequences of SEQ ID NOs:1 and 2, or complements thereof; and an oligonucleotide probe comprising or consisting of the nucleic acid sequence of SEQ ID NO:3, or a complement thereof. Another embodiment is directed to a kit for detecting one or more Malaria parasite species that may be present in a sample, the kit comprising amplification reagents comprising at least one of a DNA polymerase and nucleotide monomers, and one or more sets of oligonucleotide primers and one or more oligonucleotide probes, wherein the one or more sets of oligonucleotide primers and the one or more oligonucleotide probes comprises: a set of oligonucleotide primers comprising oligonucleotide primers comprising or consisting of the nucleic acid sequences of SEQ ID NOs:13 and 14, or complements thereof; and an oligonucleotide probe comprising or consisting of the nucleic acid sequence of SEQ ID NO:15, or a complement thereof. In another embodiment, the one or more Malaria parasite species belongs to the genus *Plasmodium.* In another embodiment, the one or more Malaria parasite species that belongs to the genus *Plasmodium* is *P. falciparum*, *P. vivax, P. ovale*, *P. malariae*, and/or *P. knowlesi.* In another embodiment, the sample is a biological sample. In another embodiment, the biological sample is whole blood, respiratory specimens, urine, fecal specimens, blood specimens, plasma, dermal swabs, nasal swabs, wound swabs, blood cultures, skin, or soft tissue infections. In another embodiment, the biological sample is whole blood. In another embodiment, at least one of the one or more oligonucleotide probes is labeled. In another embodiment, the at least one of the one or more oligonucleotide probes is labeled with a donor fluorescent moiety and a corresponding acceptor moiety.

Further provided is an oligonucleotide comprising or consisting of a sequence of nucleotides selected from SEQ ID NOs:1-39, particularly oligonucleotide comprising or consisting of a nucleic acid sequence of SEQ ID NOs:1 to 3 and 13 to 15, or complements thereof, which oligonucleotide has 100 or fewer nucleotides. The present disclosure also provides an oligonucleotide that includes a nucleic acid having at least 70% sequence identity (*e.g.*, at least 75%, 80%, 85%, 90% or 95%, etc.) to one of SEQ ID NOs:1-39, particularly oligonucleotide comprising or consisting of a nucleic acid sequence of SEQ ID NOs:1 to 3 and 13 to 15, or a complement thereof, which oligonucleotide has 100 or fewer nucleotides. Generally, these oligonucleotides as disclosed herein may be oligonucleotide primer nucleic acids, oligonucleotide probe nucleic acids, or the like. In certain aspects, the oligonucleotides have 40 or fewer nucleotides (*e.g*., 35 or fewer nucleotides, 30 or fewer nucleotides, 25 or fewer nucleotides, 20 or fewer nucleotides, 15 or fewer nucleotides, *etc.*) In some aspects, the oligonucleotides comprise at least one modified nucleotide, *e.g.*, to alter nucleic acid hybridization stability relative to unmodified nucleotides. Optionally, the oligonucleotides comprise at least one label and optionally at least one quencher moiety. In some embodiments, the oligonucleotides include at least one conservatively modified variation. "Conservatively modified variations" or, simply, "conservative variations" of a particular nucleic acid sequence refers to those nucleic acids, which encode identical or essentially identical amino acid sequences, or, where the nucleic acid does not encode an amino acid sequence, to essentially identical sequences. One of skill in the art will recognize that individual substitutions, deletions or additions which alter, add or delete a single nucleotide or a small percentage of nucleotides (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. In one aspect, amplification can employ a polymerase enzyme having 5' to 3' nuclease activity. Thus, the donor fluorescent moiety and the acceptor moiety, *e.g.*, a quencher, may be within no more than 5 to 20 nucleotides (*e.g*., within 7 or 10 nucleotides) of each other along the length of the oligonucleotide probe. In another aspect, the oligonucleotide probe includes a nucleic acid sequence that permits secondary structure formation. Such secondary structure formation may result in spatial proximity between the first and second fluorescent moiety. According to this method, the second fluorescent moiety on the oligonucleotide probe can be a quencher.

The present disclosure also provides for methods of detecting the presence or absence of Malaria parasites (such as *Plasmodium*) or Malaria parasites (such as *Plasmodium*) nucleic acid, in a biological sample from an individual. These methods can be employed to detect the presence or absence of Malaria parasites (such as *Plasmodium*) nucleic acid in plasma, for use in blood screening and diagnostic testing. Additionally, the same test may be used by someone experienced in the art to assess urine and other sample types to detect and/or quantitate Malaria parasites (such as *Plasmodium*) nucleic acid. Such methods generally include performing at least one cycling step, which includes an amplifying step and a dye-binding step. Typically, the amplifying step includes contacting the sample with a plurality of pairs of oligonucleotide primers to produce one or more amplification products if a nucleic acid molecule is present in the sample, and the dye-binding step includes contacting the amplification product with a double-stranded DNA binding dye. Such methods also include detecting the presence or absence of binding of the double-stranded DNA binding dye into the amplification product, wherein the presence of binding is indicative of the presence of Malaria parasites (such as *Plasmodium*) nucleic acid in the sample, and wherein the absence of binding is indicative of the absence of Malaria parasites nucleic acid in the sample. A representative double-stranded DNA binding dye is ethidium bromide. Other nucleic acid-binding dyes include DAPI, Hoechst dyes, PicoGreen^{®}, RiboGreen^{®}, OliGreen^{®}, and cyanine dyes such as YO-YO^{®} and SYBR^{®} Green. In addition, such methods also can include determining the melting temperature between the amplification product and the double-stranded DNA binding dye, wherein the melting temperature confirms the presence or absence of Malaria parasite (including *Plasmodium*) nucleic acid.

Also provided is a kit for detecting and/or quantitating one or more nucleic acids of Malaria parasites (such as *Plasmodium*)*.* The kit can include one or more sets of oligonucleotide primers specific for amplification of the gene target; and one or more detectable oligonucleotide probes specific for detection of the amplification products.

In one aspect, the kit can include oligonucleotide probes already labeled with donor and corresponding acceptor moieties, *e.g*., another fluorescent moiety or a dark quencher, or can include fluorophoric moieties for labeling the probes. The kit can also include nucleoside triphosphates, nucleic acid polymerase, and buffers necessary for the function of the nucleic acid polymerase. The kit can also include a package insert and instructions for using the oligonucleotide primers, oligonucleotide probes, and fluorophoric moieties to detect the presence or absence of Malaria parasites (such as *Plasmodium*) nucleic acid in a sample.

Unless otherwise defined, all technical and scientific terms used herein have the same meaning as commonly understood by one of ordinary skill in the art to which this invention belongs. Although methods and materials similar or equivalent to those described herein can be used in the practice or testing of the present subject matter, suitable methods and materials are described below. In addition, the materials, methods, and examples are illustrative only and not intended to be limiting.

The details of one or more embodiments of the invention are set forth in the accompanying drawings and the description below. Other features, objects, and advantages of the invention will be apparent from the drawings and detailed description, and from the claims.

### BRIEF DESCRIPTION OF THE FIGURES

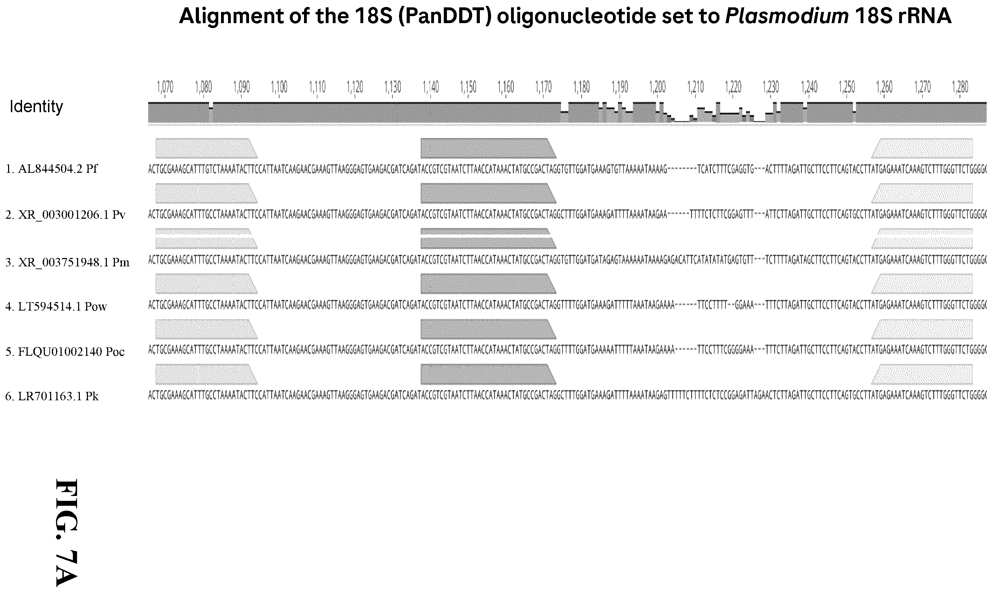

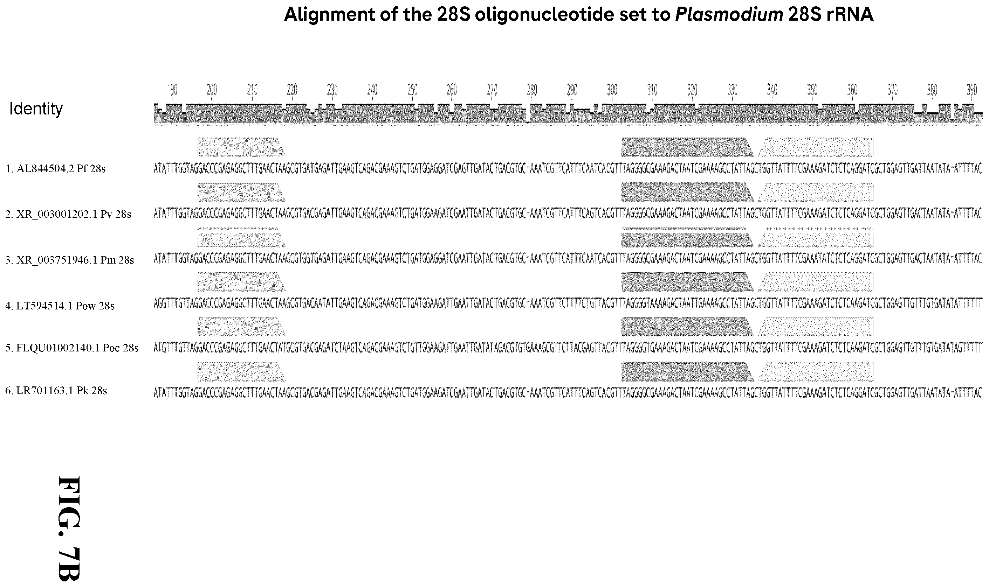

The patent or application file contains at least one drawing executed in color. Copies of this patent or patent application publication with color drawing(s) will be provided by the Office upon request and payment of the necessary fee.

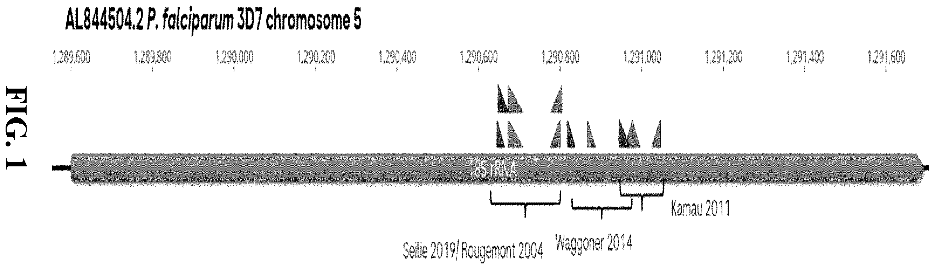

**FIG. 1** shows the initial candidate assays for an 18S rRNA target, from chromosome 5 of *P. falciparum*, as discussed in Example 1. Four different regions of the 18S rRNA genome were tested indicated by the mapped primers and probes.

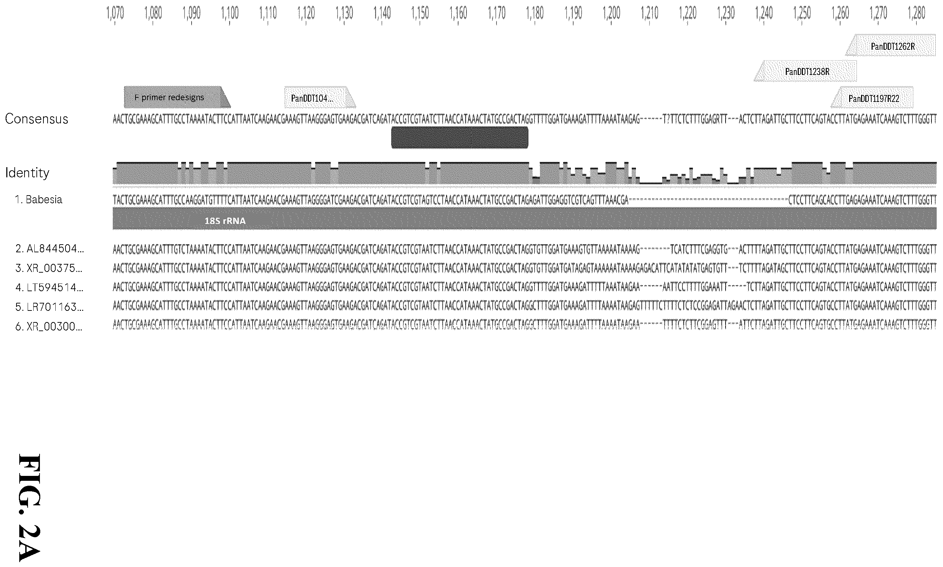

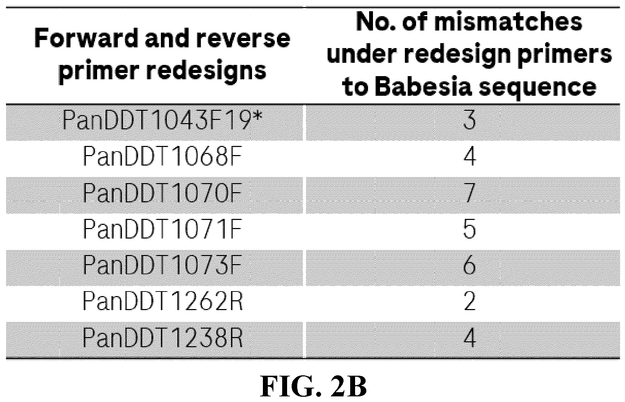

**FIG. 2A** shows an alignment map of the initial candidate primers/probes for 18S rRNA targets against *Plasmodium* species as well as *Babesia,* as described in Example 1. **FIG. 2A** shows the location of the primers and probe binding sites taken from a published PanDDT assay (Seilie, et al., "Beyond Blood Smears: Qualification of Plasmodium 18S rRNA as a Biomarker for Controlled Human Malaria Infections," Am. J. Trop. Med. Hyg. 100(6):1466-1476 (2019) (hereinafter, "Seilie, *et al.* (2019)"), and the redesigned forward primer region and reverse primers (PanDDT1238R/1262R) aligned to *Babesia* and the five *Plasmodium* species. The primers were redesigned in regions with additional mismatches (*see* table in **FIG. 2B****)** to *Babesia* to eliminate cross reactivity.

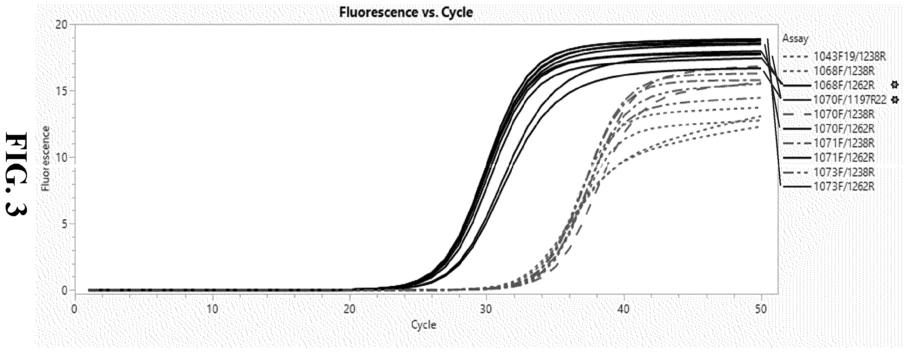

**FIG. 3** shows real time PCR growth curves of *P. falciparum* culture eluates tested with different combinations of the redesigned forward and reverse primers with the same probe in Seilie, *et al.* (2019). The forward and reverse primer in Seilie, *et al.* (2019) were also tested in combination with redesigned primers for detection of 18S rRNA targets, as described in Example 1, and as depicted in **FIG. 1****.** Some specific combinations exhibited cross reactivity to *Babesia* (designated by asterisks) and others showed delayed Ct values. These combinations were precluded from future studies. These studies demonstrate that numerous primers/probes designs for the 18S rRNA target were effective at detecting and amplifying 18S rRNA targets from *P. falciparum* culture eluates in a whole blood eluate background.

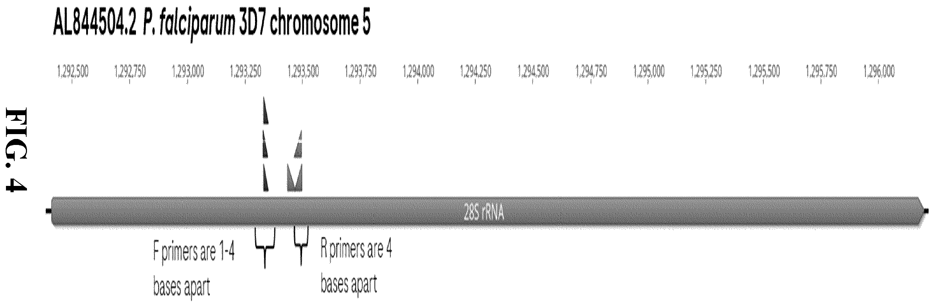

**FIG. 4** shows where the initial candidate assays for a 28S rRNA target map on chromosome 5 of *P. falciparum,* as discussed in Example 2. Combinations of three different forward primer candidates, two different reverse primer candidates, and one probe were tested, targeting the 28S rRNA.

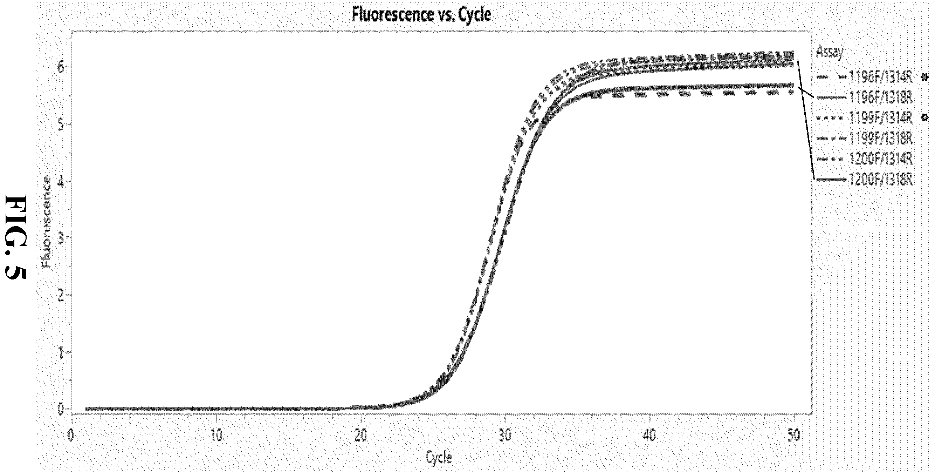

**FIG. 5** shows the real time PCR growth curves of the *P. falciparum* culture eluates tested with combinations of candidates of primers/probes for detection of 28S rRNA targets, as described in Example 2, and depicted in **FIG. 4****.** Two of the primers/probe combinations exhibited cross reactivity to *Babesia* (as indicated by asterisks). These studies demonstrate that numerous primers/probes designs targeting the 28S rRNA were effective at detecting and amplifying 28S rRNA targets from *P. falciparum* samples in a whole blood eluate background.

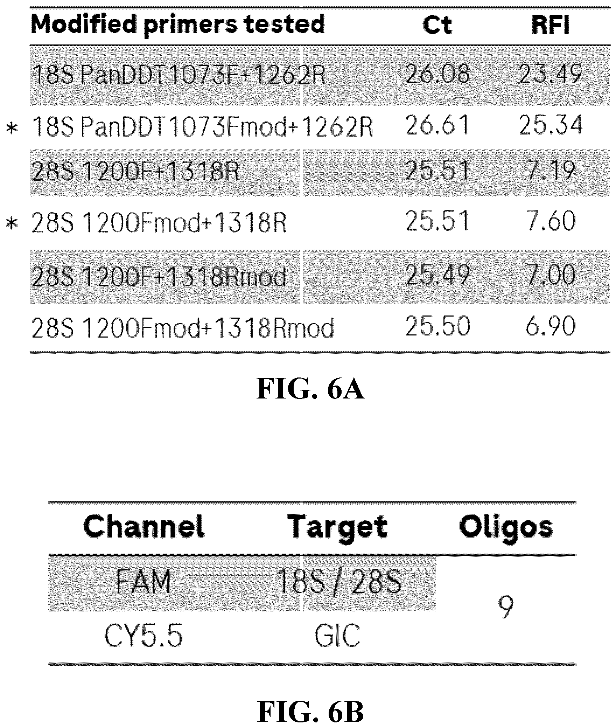

**FIG. 6A** shows results of testing modified primers of the best performing 18S and 28S candidate primers/probes for improved RFI, as described in Example 3. All combinations of the primers/probes (unmodified or modified) showed an ability to detect and amplify 18S and 28S rRNA targets from *P. falciparum* culture eluates in a whole blood background. The modified 18S and 28S single target assays exhibiting the best RFIs were designated by asterisks. **FIG. 6B** shows the *Plasmodium* 18S/28S dual-target assay design configuration, as described in Example 3. The 18S and 28S rRNA primers/probes are in the FAM channel, and the internal control is in the Cy5.5 channel. In particular, the ideal 18S rRNA target primers/probes correspond to nucleic acid sequences of SEQ ID NOs:1-3, and the ideal 28S rRNA target primer/probes correspond to nucleic acid sequences of SEQ ID NOs:13-15. It is noted that the probe used for detecting the 18S rRNA target (*i.e.*, having sequence of SEQ ID NO:3) originated from a probe for hybridizing to the 18S rRNA target from a 2019 publication (Seilie, et al., "Beyond Blood Smears: Qualification of Plasmodium 18S rRNA as a Biomarker for Controlled Human Malaria Infections," Am. J. Trop. Med. Hyg. 100(6):1466-1476 (2019)).
Collectively, **FIGs. 7A** and **7B** show the location of the primer and probe binding sites for the 18S and 28S assays mapped to each of the five *Plasmodium* species (*i.e.*, *P. falciparum* (AL844504.2), *P. vivax* (18S XR_003001206.1 and 28S XR_003001202.1), *P. malariae* (18S XR_003751948.1 and 28S XR_003751946.1), *P. ovale wallikeri* (18S and 28S LT594514.1), (*P. ovale curtisi* 18S and 28S FLQU01002140.1) and *P*. *knowlesi* (LR701163.1). For both assays, the regions under the oligonucleotides are conserved and enable detection of all five *Plasmodium* species, as described in Example 4. In particular, **FIG. 7A** shows an alignment of the candidate oligonucleotides to target the *Plasmodium* 18S rRNA region, demonstrating inclusivity of all *Plasmodium* species. **FIG. 7B** shows an alignment of the candidate oligonucleotides to target the *Plasmodium* 28S rRNA region, showing inclusivity of all *Plasmodium* species. As can be seen in **FIG. 7A** and **FIG. 7B****,** which shows alignment of the candidate dual target assay primers/probes with targets, the 18S/28S rRNA dual-target assay provides added inclusivity, as compared to a single target assay.

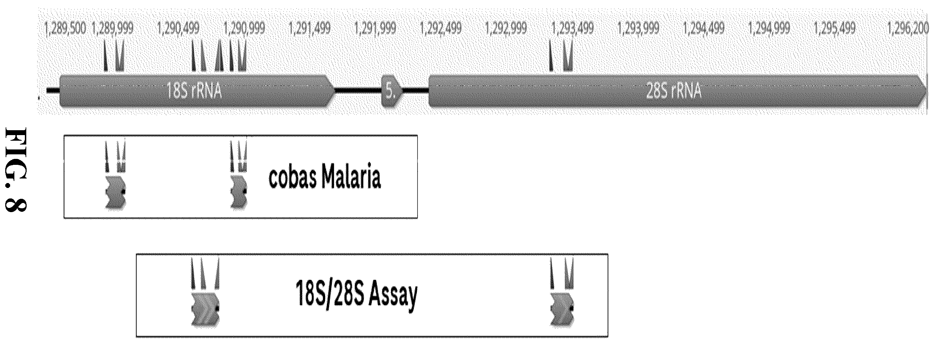

**FIG. 8** shows mapping of primers and probes for the 18S/28S dual-target *Plasmodium* assay as described in Example 4, as well as another existing Malaria assay (here labeled "cobas Malaria"), and the 18S/28S dual-target *Plasmodium* assay as described in Example 4. The existing cobas Malaria assay is a very different assay. The existing cobas Malaria assay is a dual-target assay with both targets within the 18S rRNA region. By contrast, the 18S/28S dual-target *Plasmodium* assay is a dual-target assay with the first target in the 18S rRNA region, and the second target in the 28S rRNA region.

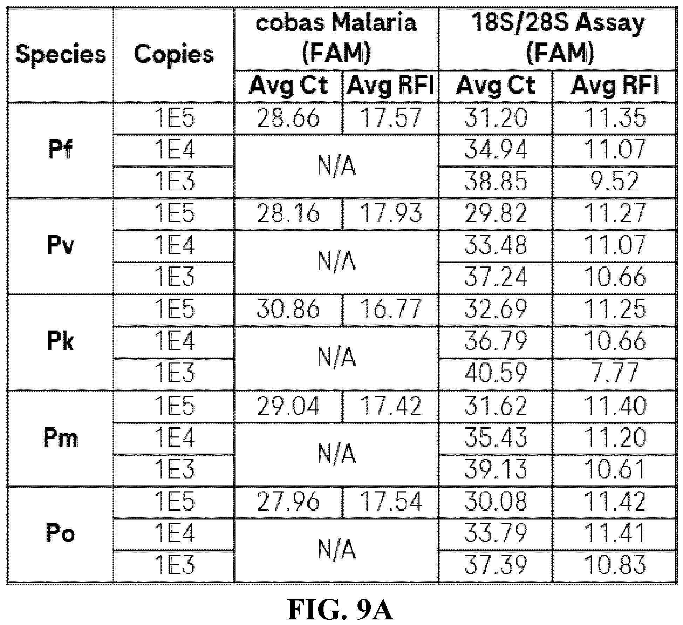

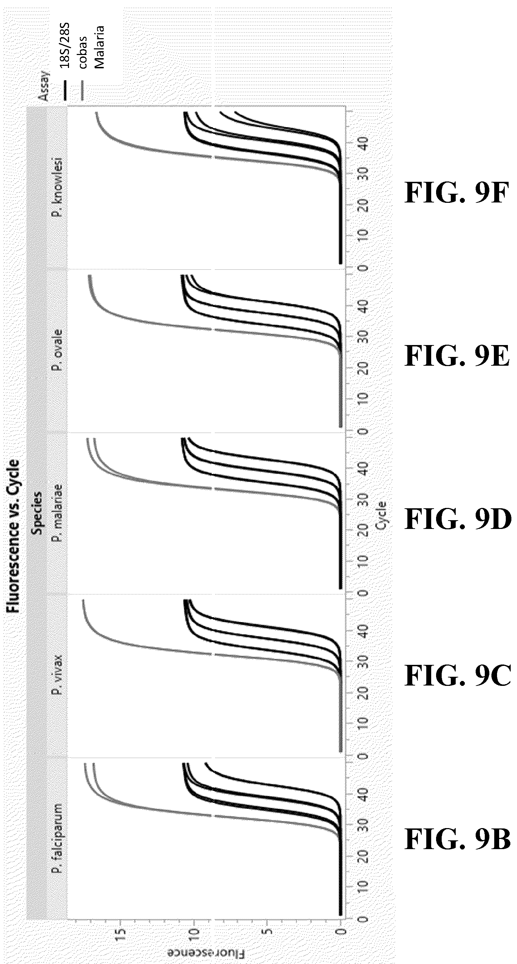

**FIGs. 9A****,** **9B, 9C, 9D, 9E,** and **9F** show that the 18S/28S dual target assay can detect all five Plasmodium species similar to the cobas Malaria assay in minigenes containing 18S and 28S rRNA targets (with primers/probe with sequences SEQ ID NOs: 1-3 for 18S rRNA targets, and with primers/probe with sequences SEQ ID NOs:13-15 for 28S rRNA targets), as described in Example 4. The cobas Malaria assay detects two targets in the 18S rRNA, while the 18S/28S dual target assay detects targets in both the 18S and 28S rRNA. In particular, **FIG. 9A** shows CT and RFI values for minigenes tested at 1E5 copies for the cobas Malaria assay and 1E5 to 1E3 copies for the 18S/28S dual target assay (with primers/probe with sequences SEQ ID NOs: 1-3 for 18S rRNA targets, and with primers/probe with sequences SEQ ID NOs: 13-15 for 28S rRNA targets) detects and amplifies all *Plasmodium* species (including *P. falciparum* (designated by the "Pf" label), *P*. *vivax* (designated by the "Pv" label), *P. ovale* (designated by the "Po" label), *P. knowlesi* (designated by the "Pk" label), and *P. malariae* (designated by the "Pm" label)) (all copy numbers were estimated using NanoDrop Microvolume Spectropotometers (ThermoFisher Scientific)). In particular, **FIG. 9B** shows the real time PCR growth curve results for *P. falciparum* minigenes amplified by the cobas Malaria (light color) and 18S/28S dual target (dark color) assays; **FIG. 9C** shows the real time PCR growth curve results for *P. vivax* minigenes amplified by the cobas Malaria (light color) and 18S/28S dual target (dark color) assays; **FIG. 9D** shows the real time PCR growth curve results for *P. malariae* minigenes amplified by the cobas Malaria (light color) and 18S/28S dual target (dark color) assays; **FIG. 9E** shows the real time PCR growth curve results for *P. ovale* minigenes amplified by the cobas Malaria (light color) and 18S/28S dual target (dark color) assays; and **FIG. 9F** shows the real time PCR growth curve results for *P. knowlesi* minigenes amplified by the cobas Malaria (light color) and 18S/28S dual target (dark color) assays. These studies demonstrate, unequivocally, that the 18S/28S rRNA dual-target *Plasmodium* assay (with primers/probes with sequences SEQ ID NOs: 1-3, for 18S rRNA targets, and primers/probes with sequences SEQ ID NOs: 13-15, for 28S rRNA targets) is able to detect the five *Plasmodium* species.

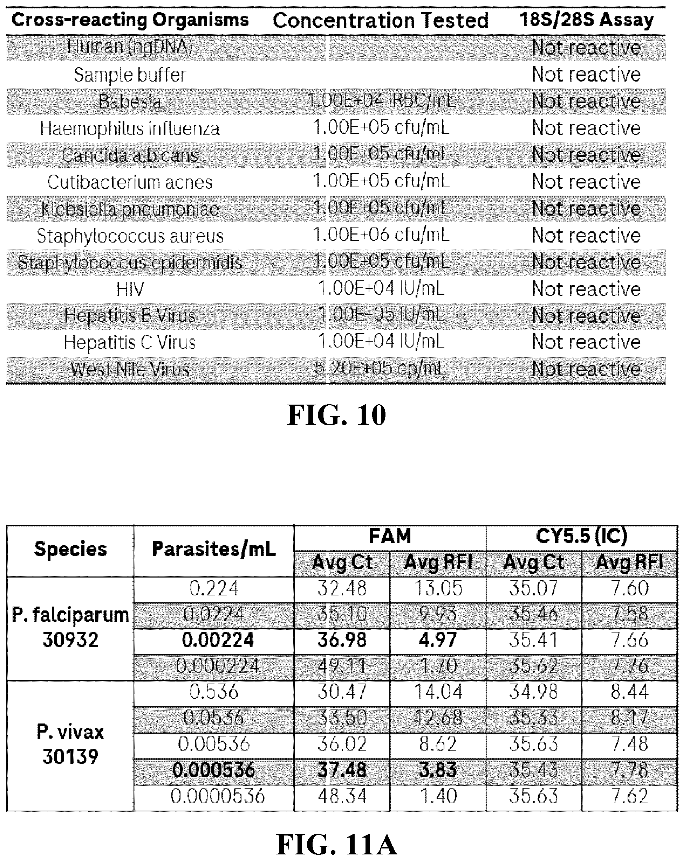

**FIG. 10** shows results from specificity testing of the 18S/28S rRNA dual-target assay with human DNA, sample buffer and some potential cross-reactive species, as described in Example 5. These studies showed that the 18S/28S rRNA dual-target assay is not reactive to any of the non-*Plasmodium* samples tested (data not shown). That is, these studies show that the 18S/28S rRNA dual-target assay is specific for *Plasmodium.* Thus, these studies demonstrate that the 18S/28S rRNA dual-target *Plasmodium* assay (with primers/probes with sequences SEQ ID NOs: 1-3, for 18S rRNA targets, and primers/probes with sequences SEQ ID NOs: 13-15, for 28S rRNA targets) is specific for *Plasmodium* species, and does not cross react with *Babesia*, *Haemophilus influenza*, *Candida albicans, Cutibacterium acnes, Klebsiella pneumoniae, Staphylococcus aureus, Staphylococcus epidermidis*, HIV, Hepatitis B Virus (HBV), Hepatitis C Virus (HCV), and West Nile Virus.

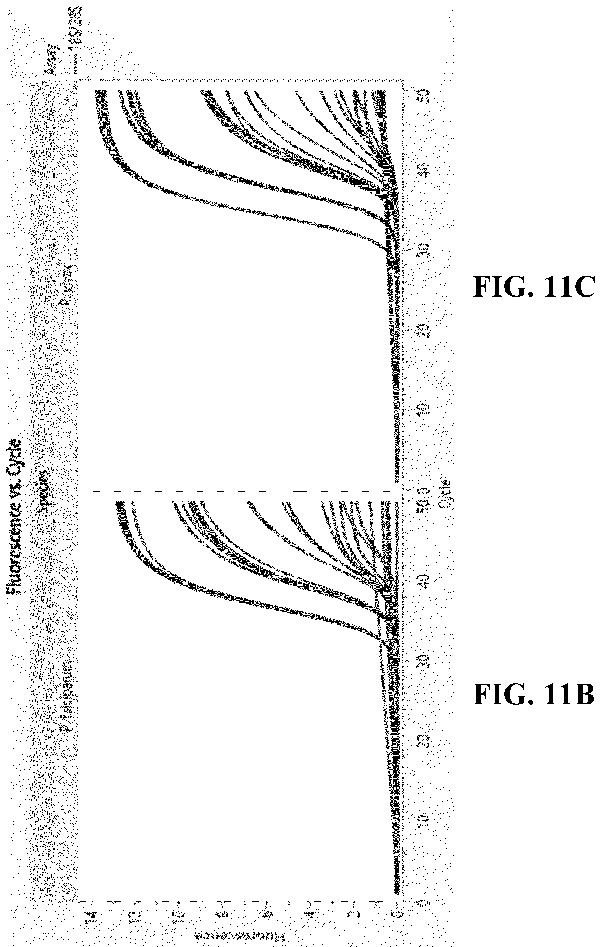

**FIGs. 11A****,** **11B,** and **11C** show the results from a sensitivity analysis of the 18S/28S rRNA dual-target *Plasmodium* assay using *P. falciparum* and *P*. *vivax* culture eluates, as described in Example 6. **FIG. 11A** depicts the data, in table format, showing that the 18S/28S rRNA dual-target assay is sensitive enough to detect 0.00224 parasites/ml for *P. falciparum* **(****FIG. 11B****),** and 0.000536 parasites/ml for *P. vivax.* **(****FIG. 11C). FIG. 11B** and **FIG. 11C** show the real time PCR growth curves for *P. falciparum* and *P. vivax* culture eluates, respectively. These studies demonstrate that the 18S/28S rRNA dual-target assay (with primers/probes with sequences SEQ ID NOs:1-3, for 18S rRNA targets, and primers/probes with sequences SEQ ID NOs:13-15, for 28S rRNA targets) is a very sensitive assay, that can detect down to one lysed *Plasmodium* parasite in the testing sample.

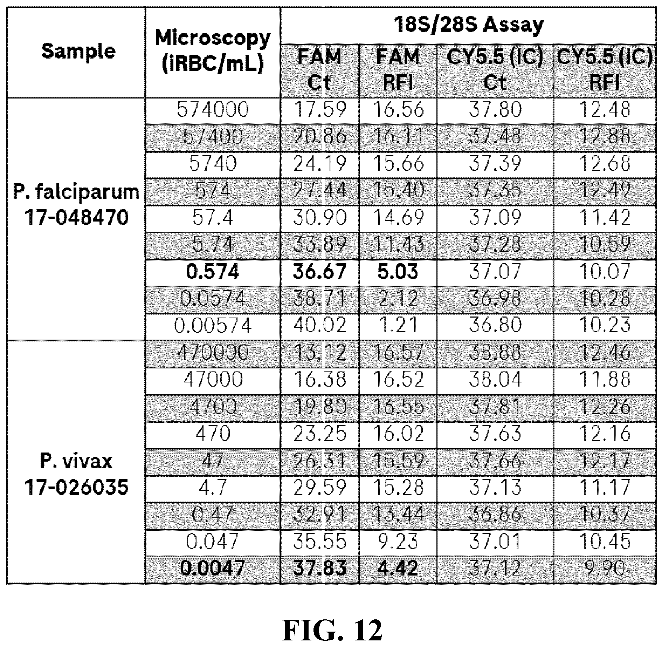

**FIG. 12** shows the results from two *Plasmodium* positive clinical samples tested with the 18S/28S rRNA dual-target *Plasmodium* assay, as described in Example 7. **FIG. 12** shows that the 18S/28S rRNA dual-target *Plasmodium* assay is able to detect *P. falciparum* and *P*. *vivax* nucleic acids from positive clinical samples. Thus, these studies demonstrate that the 18S/28S rRNA dual-target assay (with primers/probes with sequences SEQ ID NOs:1-3, for 18S rRNA targets, and primers/probes with sequences SEQ ID NOs:13-15, for 28S rRNA targets) is able to detect *Plasmodium* from clinical samples.

### DETAILED DESCRIPTION OF THE INVENTION

Diagnosis of Malaria parasites (such as *Plasmodium*) infection by nucleic acid amplification provides a method for rapidly, accurately, reliably, specifically, and sensitively detecting and/or quantitating the Malaria parasites (such as *Plasmodium*) infection. A real-time PCR assay for detecting and/or quantitating Malaria parasites nucleic acids, including DNA and/or RNA, in a non-biological or biological sample is described herein. Primers and probes for detecting and/or quantitating Malaria parasites (such as *Plasmodium*) are provided, as are articles of manufacture or kits containing such primers and probes. The increased specificity and sensitivity of real-time PCR for detection of Malaria parasites (such as *Plasmodium*) compared to other methods, as well as the improved features of real-time PCR including sample containment and real-time detection and quantitating of the amplified product, make feasible the implementation of this technology for routine diagnosis of Malaria parasites (such as *Plasmodium*) infections in the clinical laboratory. Additionally, this technology may be employed for blood screening as well as for prognosis. This Malaria parasites (such as *Plasmodium*) detection assay may also be multiplexed with other assays for the detection of other nucleic acids, *e.g.,* other bacteria and/or viruses, in parallel.

Thus, the assay for detection of Malaria parasites is a qPCR-based assay was developed to detect the *Plasmodium* species: *P. falciparum*, *P. vivax*, *P. ovale*, *P. knowlesi*, and *P. malariae*, which cause malaria, in blood samples. This qPCR assay was designed for the cobas 6800/8800 platform. Although the Unites States is considered a non-endemic country, global travel may lead to an increase in the cases of imported malaria, thereby, affecting the blood supply. There are two strategies regarding malaria that are employed to maintain safety of blood supplies: (1) Selective Testing, and (2) Donor Deferral. Currently, the United States has a donor deferral policy for blood donations, where donors are deferred based on questionnaire answers regarding their history of malaria infection, prior residency of an endemic country, and travel to an endemic country. Donor deferrals negatively impact the blood supply by excluding donations based on questionnaire answers, regardless of malaria status. On the other hand, selective testing reduces the impact on blood supply inventory as compared to donor deferral. Selective testing of blood donations is also based on questionnaire; however, the selective testing policy allows for shorter deferral times and reinstatement of donors depending on the donor's malaria status. In countries that have the selective testing policy, testing is done via enzyme immunoassays. There are, at present, no IVD nucleic acid tests that are available for screening blood donations. In comparison to enzyme immunoassays, nucleic acid tests are more sensitivity at detecting malaria parasites in blood. The assay's design strategy for the qPCR based test was to target genes that are present in multiple copies within the cell. This test targets 28S and 18S ribosomal RNA. Detection of multiple copy DNA and RNA targets in parallel allows for increased sensitivity. This qPCR assay can be used by hospitals and blood centers to screen for malaria in collected blood donations.

The present disclosure includes oligonucleotide primers and fluorescent labeled hydrolysis probes that hybridize to the Malaria parasites (such as *Plasmodium*) genome, in order to specifically identify Malaria parasites (such as *Plasmodium*) using, *e.g.*, TaqMan^{®} amplification and detection technology.

The disclosed methods may include performing at least one cycling step that includes amplifying one or more portions of the nucleic acid molecule gene target from a sample using one or more pairs of primers. *"Plasmodium* primer(s)" as used herein refer to oligonucleotide primers that specifically anneal to nucleic acid sequences found in the Malaria parasites (such as *Plasmodium*) genome, and initiate DNA synthesis therefrom under appropriate conditions producing the respective amplification products. Examples of nucleic acid sequences found in the *Plasmodium* species and genome include targets such as nucleic acids within the Mitochondrial DNA target area (MT-1 and MT-2), RNA repeat sequences R125, and 18S Ribosomal RNA (18S rRNA) and 28S Ribosomal RNA (28S rRNA). Each of the discussed *Plasmodium* primers anneals to a target such that at least a portion of each amplification product contains nucleic acid sequence corresponding to the target. The one or more amplification products are produced provided that one or more nucleic acid is present in the sample, thus the presence of the one or more amplification products is indicative of the presence of *Plasmodium* in the sample. The amplification product should contain the nucleic acid sequences that are complementary to one or more detectable probes for *Plasmodium. "Plasmodium* probe(s)" as used herein refer to oligonucleotide probes that specifically anneal to nucleic acid sequences found in the *Plasmodium* genome. Each cycling step includes an amplification step, a hybridization step, and a detection step, in which the sample is contacted with the one or more detectable *Plasmodium* probes for detection of the presence or absence of *Plasmodium* in the sample.

As used herein, the term "amplifying" refers to the process of synthesizing nucleic acid molecules that are complementary to one or both strands of a template nucleic acid molecule (e.g., nucleic acid molecules from the *Plasmodium* genome). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (*e.g*., Platinum^{®} Taq) and an appropriate buffer and/or co-factors for optimal activity of the polymerase enzyme (*e.g*., MgCl₂ and/or KCl).

The term "primer" as used herein is known to those skilled in the art and refers to oligomeric compounds, primarily to oligonucleotides but also to modified oligonucleotides that are able to "prime" DNA synthesis by a template-dependent DNA polymerase, *i.e.*, the 3'-end of the, *e.g.*, oligonucleotide provides a free 3'-OH group where further "nucleotides" may be attached by a template-dependent DNA polymerase establishing 3' to 5' phosphodiester linkage whereby deoxynucleoside triphosphates are used and whereby pyrophosphate is released.

The term "hybridizing" refers to the annealing of one or more probes to an amplification product. "Hybridization conditions" typically include a temperature that is below the melting temperature of the probes but that avoids non-specific hybridization of the probes.

The term "5' to 3' nuclease activity" refers to an activity of a nucleic acid polymerase, typically associated with the nucleic acid strand synthesis, whereby nucleotides are removed from the 5' end of nucleic acid strand.

The term "thermostable polymerase" refers to a polymerase enzyme that is heat stable, *i.e.*, the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus*, *T. ruber*, *T. thermophilus*, *T. aquaticus*, *T. lacteus*, *T. rubens*, *Bacillus stearothermophilus*, and *Methanothermus fervidus.* Nonetheless, polymerases that are not thermostable also can be employed in PCR assays provided the enzyme is replenished, if necessary.

The term "complement thereof" refers to nucleic acid that is both the same length as, and exactly complementary to, a given nucleic acid.

The term "extension" or "elongation" when used with respect to nucleic acids refers to when additional nucleotides (or other analogous molecules) are incorporated into the nucleic acids. For example, a nucleic acid is optionally extended by a nucleotide incorporating biocatalyst, such as a polymerase that typically adds nucleotides at the 3' terminal end of a nucleic acid.

The terms "identical" or percent "identity" in the context of two or more nucleic acid sequences, refer to two or more sequences or subsequences that are the same or have a specified percentage of nucleotides that are the same, when compared and aligned for maximum correspondence, *e.g*., as measured using one of the sequence comparison algorithms available to persons of skill or by visual inspection. Exemplary algorithms that are suitable for determining percent sequence identity and sequence similarity are the BLAST programs, which are described in, *e.g.,* Altschul et al. (1990) "Basic local alignment search tool" J. Mol. Biol. 215:403-410, Gish et al. (1993) "Identification of protein coding regions by database similarity search" Nature Genet. 3:266-272, Madden et al. (1996) "Applications of network BLAST server" Meth. Enzymol. 266:131-141, Altschul et al. (1997) "Gapped BLAST and PSI-BLAST: a new generation of protein database search programs" Nucleic Acids Res. 25:3389-3402, and Zhang et al. (1997) "PowerBLAST: A new network BLAST application for interactive or automated sequence analysis and annotation" Genome Res. 7 649-656.

A "modified nucleotide" in the context of an oligonucleotide refers to an alteration in which at least one nucleotide of the oligonucleotide sequence is replaced by a different nucleotide that provides a desired property to the oligonucleotide. Exemplary modified nucleotides that can be substituted in the oligonucleotides described herein include, *e.g.*, a t-butyl benzyl, a C5-methyl-dC, a C5-ethyl-dC, a C5-methyl-dU, a C5-ethyl-dU, a 2,6-diaminopurine, a C5-propynyl-dC, a C5-propynyl-dU, a C7-propynyl-dA, a C7-propynyl-dG, a C5-propargylamino-dC, a C5-propargylamino-dU, a C7-propargylamino-dA, a C7-propargylamino-dG, a 7-deaza-2-deoxyxanthosine, a pyrazolopyrimidine analog, a pseudo-dU, a nitro pyrrole, a nitro indole, 2'-0-methyl ribo-U, 2'-0-methyl ribo-C, an N4-ethyl-dC, an N6-methyl-dA, a 5-propynyl dU, a 5-propynyl dC, 7-deaza-deoxyguanosine (deaza G (u-deaza)) and the like. Many other modified nucleotides that can be substituted in the oligonucleotides are referred to herein or are otherwise known in the art. In certain embodiments, modified nucleotide substitutions modify melting temperatures (Tm) of the oligonucleotides relative to the melting temperatures of corresponding unmodified oligonucleotides. To further illustrate, certain modified nucleotide substitutions can reduce non-specific nucleic acid amplification (*e.g*., minimize primer dimer formation or the like), increase the yield of an intended target amplicon, and/or the like in some embodiments. Examples of these types of nucleic acid modifications are described in, *e.g.,* U.S. Patent No. 6,001,611.

Other modified nucleotide substitutions may alter the stability of the oligonucleotide, or provide other desirable features.

### Detection/Quantitation of Malaria Parasite (including Plasmodium) Target Nucleic Acid

The present disclosure provides methods to detect Malaria parasites (including *Plasmodium*) by amplifying, for example, a portion of the *Plasmodium* nucleic acid sequence. Specifically, primers and probes to amplify and detect and/or quantitate *Plasmodium* nucleic acid molecule targets are provided by the embodiments in the present disclosure.

For detection and/or quantitation of Malaria parasites (including *Plasmodium*), primers and probes to amplify and detect/quantitate the *Plasmodium* are provided. *Plasmodium* nucleic acids other than those exemplified herein can also be used to detect *Plasmodium* in a sample. For example, functional variants can be evaluated for specificity and/or sensitivity by those of skill in the art using routine methods. Representative functional variants can include, *e.g.*, one or more deletions, insertions, and/or substitutions in the *Plasmodium* nucleic acids disclosed herein.

More specifically, embodiments of the oligonucleotides each include a nucleic acid with a sequence selected from SEQ ID NOs:1-39, particularly oligonucleotide comprising or consisting of a nucleic acid sequence of SEQ ID NOs:1 to 3 and 13 to 15, a substantially identical variant thereof in which the variant has at least, *e.g.*, 80%, 90%, or 95% sequence identity to one of SEQ ID NOs: 1-39, or a complement of SEQ ID NOs:1-39 and the variant. Table 1, below, shows the oligonucleotides (primers and probes) used in a Plasmodium assay for an 18S rRNA target.

**Table 1: Oligonucleotides (primers and probes) used in Plasmodium assay (18S rRNA Target)**

| **Type** | **Oligo ID** | **SEQ ID.** | **Sequence** |
|---|---|---|---|
| F. Primer | PanDDT1073FMO D | 1 | TGCGAAAGCATTTGCCTAAAATACTT<t_BB_dC> |
| | | 27 | TGCGAAAGCATTTGCCTAAAATACTTC |
| R. Primer | PanDDT1262R | 2 | CAGAACCCAAAGACTTTGATTTCTCAT |
| Probe | PanDDTprobe | 3 | <FAM_Thr>ACCGT<BHQ_2>CGTAATCTTAACCATAAACT ATGCCGACTAG<Spc_C3> |
| | | 28 | ACCGTCGTAATCTTAACCATAAACTATGCCGACTAG |
| F. Primer | PanDDT1073F | 4 | TGCGAAAGCATTTGCCTAAAATACTTC |
| F. Primer | PanDDT1043F19 | 5 | AAAGTTAAGGGAGTGAAGA |
| R. Primer | PanDDT1197R22 | 6 | AAGACTTTGATTTCTCATAAGG |
| F. Primer | PanDDT1068F | 7 | ACAACTGCGAAAGCATTTGCCTAAA |
| F. Primer | PanDDT1068Fmod | 8 | ACAACTGCGAAAGCATTTGCCTAA<t BB dA> |
| | | 29 | ACAACTGCGAAAGCATTTGCCTAAA |
| F. Primer | PanDDT1070F | 9 | AACTGCGAAAGCATTTGTCTAAAATACTTC |
| F. Primer | PanDDT1071F | 10 | ACTGCGAAAGCATTTGCCTAAAATAC |
| F. Primer | PanDDT1071Fmod | 11 | ACTGCGAAAGCATTTGCCTAAAATA<t_BB_dC> |
| | | 30 | ACTGCGAAAGCATTTGCCTAAAATAC |
| R. Primer | PanDDT1238R | 12 | CATAAGGTACTGAAGGAAGCAATCTAA |

Table 2, below, shows the oligonucleotides (primer and probes) used in the *Plasmodium* assay for a 28S rRNA target.

**Table 2: Oligonucleotides (primers and probes) used in Plasmodium assay (28S rRNA Target)**

| **Type** | **Oligo ID** | **SEQ ID.** | **Sequence** |
|---|---|---|---|
| F. Primer | 28S_1179_1200Fm od | 13 | GACCCGAGAGGCTTTGAACTA<t_BB_dA> |
| | | 31 | GACCCGAGAGGCTTTGAACTAA |
| R. Primer | 28S_1346_1318R | 14 | GATCCTGAGAGATCTTTCGAAAATAACCA |
| Probe | 28S_1284_1316P1 | 15 | <FAM_Thr>TAGGG<BHQ_2>GCGAAAGA<pdC><pdU> AA<pdU><pdC>GAAAAG<pdC><pdC><pdU>ATTAG<S pc_C3> |
| | | 32 | TAGGGGCGAAAGACTAATCGAAAAGCCTATTAG |
| F. Primer | 28S_1179_1200F | 16 | GACCCGAGAGGCTTTGAACTAA |
| R. Primer | 28S_1346_1318Rm od | 17 | GATCCTGAGAGATCTTTCGAAAATAACC<t_BB_dA> |
| | | 33 | GATCCTGAGAGATCTTTCGAAAATAACCA |
| F. Primer | 28S_1178_1196F | 18 | GGACCCGAGAGGCTTTGAA |
| F. Primer | 28S_1178_1196Fm od | 19 | GGACCCGAGAGGCTTTGA<t BB dA> |
| | | 34 | GGACCCGAGAGGCTTTGAA |
| F. Primer | 28S_1179_1199F | 20 | GACCCGAGAGGCTTTGAACTA |
| F. Primer | 28S_1179_1199Fm od | 21 | GACCCGAGAGGCTTTGAACT<t BB dA> |
| | | 35 | GACCCGAGAGGCTTTGAACTA |
| R. Primer | 28S_1343_1314R | 22 | CCTGAGAGATCTTTCGAAAATAACCAGCTA |
| R. Primer | 28S_1343_1314Rm od | 23 | CCTGAGAGATCTTTCGAAAATAACCAGCT<t BB dA> |
| | | 36 | CCTGAGAGATCTTTCGAAAATAACCAGCTA |
| Probe | 28S_1284_1316P2 | 24 | <FAM_Thr>TAGGG<BHQ_2>GTGAAGAA<pdU><pdC> ATACGAAAG<pdC><pdC><pdU>AATAG<Spc_C3> |
| | | 37 | TAGGGGTGAAGAATCATACGAAAAGCCTAATAG |
| Probe | 28S_1284_1316P3 | 25 | <FAM_Thr>TAGGG<BHQ_2>GTGAAAGA<pdC><pdC> A<pdU>ACGAAAAG<pdC><pdC><pdU>AATAG<Spc_C 3> |
| | | 38 | TAGGGGTGAAAGACCATACGAAAAGCCTAATAG |
| Probe | 28S_1284_1316P4 | 26 | <FAM_Thr>TAGGG<BHQ_2>GTAAAAGA<pdC><pdU> AATTGAAAAG<pdC><pdC><pdU>A<pdU><pdU>AG<S pc_C3> |
| | | 39 | TAGGGGTAAAAGACTAATTGAAAAGCCTATTAG |

In one embodiment, the above-described sets of *Plasmodium* primers and probes are used in order to provide for detection of Malaria parasites (including *Plasmodium*) in a biological sample suspected of containing *Plasmodium,* targeting the 18S rRNA (Table 1). In one embodiment, the above described sets of *Plasmodium* primers and probes are used in order to provide for detection of Malaria parasites (including *Plasmodium*) in a biological sample suspected of containing *Plasmodium,* targeting the 28S rRNA (Table 2).

In one embodiment, the assay is a dual target assay, with a first target being the 18S rRNA (using oligonucleotides from Table 1), and with a second target being the 28S rRNA (using oligonucleotides from Table 2). In such a dual target assay, two targets (18S rRNA and 28S rRNA) are being detected simultaneously in the same sample. Ribosomal RNA targets were selected because they are present in multiple copies. Furthermore, rRNA sequences on different chromosomes, while not identical, are conserved. In general, an assay targeting more than one target, such as a dual target (or duplex assay), is advantageous over an assay that targets just a single target (or singleplex assay). In particular, detection of multiple copy targets in parallel allows for increased sensitivity. Furthermore, detection of multiple copy targets in parallel, also mitigates the risk of any one singleplex assay failing by covering multiple targets. Thus, the dual target assay provided herein, represents an improvement over the art, particularly the art of single target assays.

The sets of primers and probes may comprise or consist of the primers and probes specific for the nucleic acid sequences of Malaria parasites (including *Plasmodium*), comprising or consisting of the nucleic acid sequences of SEQ ID NOs: 1-39, particularly comprising or consisting of a nucleic acid sequence of SEQ ID NOs: 1 to 3 and 13 to 15. In another embodiment, the primers and probes for the *Plasmodium* target comprise or consist of a functionally active variant of any of the primers and probes of SEQ ID NOs: 1-39, particularly comprising or consisting of a nucleic acid sequence of SEQ ID NOs: 1 to 3 and 13 to 15.

A functionally active variant of any of the primers and/or probes of SEQ ID NOs:1-39 may be identified by using the primers and/or probes in the disclosed methods. A functionally active variant of a primer and/or probe of any of the SEQ ID NOs:1-39 pertains to a primer and/or probe which provide a similar or higher specificity and sensitivity in the described method or kit as compared to the respective sequence of SEQ ID NOs: 1-39.

The variant may, *e.g*., vary from the sequence of SEQ ID NOs:1-39 by one or more nucleotide additions, deletions or substitutions such as one or more nucleotide additions, deletions or substitutions at the 5' end and/or the 3' end of the respective sequence of SEQ ID NOs:1-39. As detailed above, a primer and/or probe may be chemically modified, *i.e.*, a primer and/or probe may comprise a modified nucleotide or a non-nucleotide compound. A probe (or a primer) is then a modified oligonucleotide. "Modified nucleotides" (or "nucleotide analogs") differ from a natural "nucleotide" by some modification but still consist of a base or base-like compound, a pentofuranosyl sugar or a pentofuranosyl sugar-like compound, a phosphate portion or phosphate-like portion, or combinations thereof. For example, a "label" may be attached to the base portion of a "nucleotide" whereby a "modified nucleotide" is obtained. A natural base in a "nucleotide" may also be replaced by, *e.g.*, a 7-desazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

Oligonucleotides including modified oligonucleotides and oligonucleotide analogs that amplify a nucleic acid molecule encoding the *Plasmodium* target, *e.g.*, nucleic acids encoding alternative portions of *Plasmodium* can be designed using, for example, a computer program such as OLIGO (Molecular Biology Insights Inc., Cascade, Colo.). Important features when designing oligonucleotides to be used as amplification primers include, but are not limited to, an appropriate size amplification product to facilitate detection (*e.g*., by electrophoresis), similar melting temperatures for the members of a pair of primers, and the length of each primer (*i.e.*, the primers need to be long enough to anneal with sequence-specificity and to initiate synthesis but not so long that fidelity is reduced during oligonucleotide synthesis). Typically, oligonucleotide primers are 8 to 50 nucleotides in length (*e.g.*, 8, 10, 12, 14, 16, 18, 20, 22, 24, 26, 28, 30, 32, 34, 36, 38, 40, 42, 44, 46, 48, or 50 nucleotides in length). The disclosed primers for detection and amplification of Malaria parasites (including *Plasmodium*) include SEQ ID NOs: 1, 2, 4-23, 27, 29, and 30-36.

In addition to a set of primers, the methods may use one or more probes in order to detect the presence or absence of *Plasmodium.* The term "probe" refers to synthetically or biologically produced nucleic acids (DNA or RNA), which by design or selection, contain specific nucleotide sequences that allow them to hybridize under defined predetermined stringencies specifically (*i.e*., preferentially) to "target nucleic acids", in the present case to a *Plasmodium* (target) nucleic acid. A "probe" can be referred to as a "detection probe" meaning that it detects the target nucleic acid. In some embodiments, the described *Plasmodium* probes can be labeled with at least one fluorescent label. In one embodiment, the *Plasmodium* probes can be labeled with a donor fluorescent moiety, *e.g.,* a fluorescent dye, and a corresponding acceptor moiety, *e.g.,* a quencher. In one embodiment, the probe comprises or consists of a fluorescent moiety and the nucleic acid sequences comprise or consist of SEQ ID NOs:3, 24-26, 28, and 37-39. The disclosed probes for detection of Malaria parasites (including *Plasmodium*), via, for example, hybridization/annealing to amplicons, include SEQ ID NOs:3, 24-26, 28, and 37-39.

Designing oligonucleotides to be used as probes can be performed in a manner similar to the design of primers. Embodiments may use a single probe or a pair of probes for detection of the amplification product. Depending on the embodiment, the probe(s) use may comprise at least one label and/or at least one quencher moiety. As with the primers, the probes usually have similar melting temperatures, and the length of each probe must be sufficient for sequence-specific hybridization to occur but not so long that fidelity is reduced during synthesis. Oligonucleotide probes are generally 15 to 40 (*e.g.*, 15, 16, 18, 20, 21, 22, 23, 24, 25, 26, 27, 28, 29, 30, 31, 32, 33, 34, 35, 36, 37, 39, or 40) nucleotides in length.

Constructs can include vectors each containing one of *Plasmodium* primers and probes nucleic acid molecules (*e.g*., SEQ ID NOs:1-39). Constructs can be used, for example, as control template nucleic acid molecules. Vectors suitable for use are commercially available and/or produced by recombinant nucleic acid technology methods routine in the art. *Plasmodium* nucleic acid molecules can be obtained, for example, by chemical synthesis, direct cloning from *Plasmodium*, or by nucleic acid amplification.

Constructs suitable for use in the methods typically include, in addition to the *Plasmodium* nucleic acid molecules, and/or primers/probes for amplification and/or detection of *Plasmodium* (*e.g.*, *a* nucleic acid molecule that contains one or more sequences of SEQ ID NOs:1-39), sequences encoding a selectable marker (*e.g.*, an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs containing *Plasmodium* nucleic acid molecules, and/or primers/probes for amplification and/or detection of *Plasmodium,* can be propagated in a host cell. As used herein, the term host cell is meant to include prokaryotes and eukaryotes such as yeast, plant and animal cells. Prokaryotic hosts may include *E. coli*, *Salmonella typhimurium*, *Serratia marcescens*, and *Bacillus subtilis.* Eukaryotic hosts include yeasts such as *S. cerevisiae*, *S. pombe*, *Pichia pastoris*, mammalian cells such as COS cells or Chinese hamster ovary (CHO) cells, insect cells, and plant cells such as *Arabidopsis thaliana* and *Nicotiana tabacum.* A construct can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, *e.g.,* U.S. Patent Nos. 5,580,859 and 5,589,466).

### Polymerase Chain Reaction (PCR)

U.S. Patent Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in some embodiments include oligonucleotides capable of acting as points of initiation of nucleic acid synthesis within the described *Plasmodium* nucleic acid sequences (e.g., SEQ ID NOs: 1, 2, 4-23, 27, 29, and 30-36). A primer can be purified from a restriction digest by conventional methods, or it can be produced synthetically. The primer is preferably single-stranded for maximum efficiency in amplification, but the primer can be double-stranded. Double-stranded primers are first denatured, *i.e*., treated to separate the strands. One method of denaturing double stranded nucleic acids is by heating.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, *e.g.,* on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (*e.g.*, 1 min to 2 min 30 sec, or 1.5 min).

If the double-stranded template nucleic acid is denatured by heat, the reaction mixture is allowed to cool to a temperature that promotes annealing of each primer to its target sequence. The temperature for annealing is usually from about 35°C to about 65°C (*e.g.*, about 40°C to about 60°C; about 45°C to about 50°C). Annealing times can be from about 10 sec to about 1 min (*e.g.*, about 20 sec to about 50 sec; about 30 sec to about 40 sec). The reaction mixture is then adjusted to a temperature at which the activity of the polymerase is promoted or optimized, *i.e.*, a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (*e.g.*, the temperature for extension generally ranges from about 40°C to about 80°C (*e.g.*, about 50°C to about 70°C; about 60°C). Extension times can be from about 10 sec to about 5 min (*e.g*., about 30 sec to about 4 min; about 1 min to about 3 min; about 1 min 30 sec to about 2 min).

The genome of a retrovirus or RNA virus is comprised of a ribonucleic acid, *i.e.*, RNA. In such case, the template nucleic acid, RNA, must first be transcribed into complementary DNA (cDNA) via the action of the enzyme reverse transcriptase. Reverse transcriptases use an RNA template and a short primer complementary to the 3' end of the RNA to direct synthesis of the first strand cDNA, which can then be used directly as a template for polymerase chain reaction.

PCR assays can employ *Plasmodium* nucleic acid, and/or primers/probes that amplify and/or detect *Plasmodium,* such as RNA or DNA (cDNA). The template nucleic acid need not be purified; it may be a minor fraction of a complex mixture, such as *Plasmodium* nucleic acid contained in human cells. *Plasmodium* nucleic acid molecules, and/or primers/probes that amplify and/or detect *Plasmodium* may be extracted from a biological sample by routine techniques such as those described in Diagnostic Molecular Microbiology: Principles and Applications (Persing, et al. (eds), 1993, American Society for Microbiology, Washington D.C.). Nucleic acids can be obtained from any number of sources, such as plasmids, or natural sources including bacteria, yeast, viruses, organelles, or higher organisms such as plants or animals.

The oligonucleotide primers (*e.g*., SEQ ID NOs:1, 2, 4-23, 27, 29, and 30-36) are combined with PCR reagents under reaction conditions that induce primer extension. For example, chain extension reactions generally include 50 mM KCl, 10 mM Tris-HCl (pH 8.3), 15 mM MgCl₂, 0.001% (w/v) gelatin, 0.5-1.0 µg denatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly-synthesized strands form a double-stranded molecule that can be used in the succeeding steps of the reaction. The steps of strand separation, annealing, and elongation can be repeated as often as needed to produce the desired quantity of amplification products corresponding to the target nucleic acid molecules of Malaria parasites (including *Plasmodium*)*.* The limiting factors in the reaction are the amounts of primers, thermostable enzyme, and nucleoside triphosphates present in the reaction. The cycling steps (*i.e.*, denaturation, annealing, and extension) are preferably repeated at least once. For use in detection, the number of cycling steps will depend, *e.g.*, on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence Resonance Energy Transfer (FRET)

FRET technology (see, for example, U.S. Patent Nos. 4,996,143, 5,565,322, 5,849,489, and 6,162,603) is based on a concept that when a donor fluorescent moiety and a corresponding acceptor fluorescent moiety are positioned within a certain distance of each other, energy transfer takes place between the two fluorescent moieties that can be visualized or otherwise detected and/or quantitated. The donor typically transfers the energy to the acceptor when the donor is excited by light radiation with a suitable wavelength. The acceptor typically re-emits the transferred energy in the form of light radiation with a different wavelength. In certain systems, non-fluorescent energy can be transferred between donor and acceptor moieties, by way of biomolecules that include substantially non-fluorescent donor moieties (see, for example, US Patent. No. 7,741,467).

In one example, an oligonucleotide probe can contain a donor fluorescent moiety or dye (*e.g.*, HEX or FAM) and a corresponding quencher (e.g., BlackHole Quencher^{™} (BHQ) (such as BHQ-2)), which may or not be fluorescent, and which dissipates the transferred energy in a form other than light. When the probe is intact, energy transfer typically occurs between the donor and acceptor moieties such that fluorescent emission from the donor fluorescent moiety is quenched the acceptor moiety. During an extension step of a polymerase chain reaction, a probe bound to an amplification product is cleaved by the 5' to 3' nuclease activity of, *e.g.*, a Taq Polymerase such that the fluorescent emission of the donor fluorescent moiety is no longer quenched. Exemplary probes for this purpose are described in, *e.g.*, U.S. Patent Nos. 5,210,015, 5,994,056, and 6,171,785. Commonly used donor-acceptor pairs include the FAM-TAMRA pair. Commonly used quenchers are DABCYL and TAMRA. Commonly used dark quenchers include BlackHole Quencher^{™} (BHQ) (such as BHQ2), (Biosearch Technologies, Inc., Novato, Cal.), Iowa Black^{™}, (Integrated DNA Tech., Inc., Coralville, Iowa), BlackBerry^{™} Quencher 650 (BBQ-650), (Berry & Assoc., Dexter, Mich.).

In another example, two oligonucleotide probes, each containing a fluorescent moiety, can hybridize to an amplification product at particular positions determined by the complementarity of the oligonucleotide probes to the *Plasmodium* target nucleic acid sequence. Upon hybridization of the oligonucleotide probes to the amplification product nucleic acid at the appropriate positions, a FRET signal is generated. Hybridization temperatures can range from about 35°C to about 65°C for about 10 sec to about 1 min.

Fluorescent analysis can be carried out using, for example, a photon counting epifluorescent microscope system (containing the appropriate dichroic mirror and filters for monitoring fluorescent emission at the particular range), a photon counting photomultiplier system, or a fluorimeter. Excitation to initiate energy transfer, or to allow direct detection of a fluorophore, can be carried out with an argon ion laser, a high intensity mercury (Hg) arc lamp, a xenon lamp, a fiber optic light source, or other high intensity light source appropriately filtered for excitation in the desired range.

As used herein with respect to donor and corresponding acceptor moieties "corresponding" refers to an acceptor fluorescent moiety or a dark quencher having an absorbance spectrum that overlaps the emission spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Foerster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, helium-cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC Red 640, LC Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate, or other chelates of Lanthanide ions (e.g., Europium, or Terbium). Donor and acceptor fluorescent moieties can be obtained, for example, from Molecular Probes (Junction City, Oreg.) or Sigma Chemical Co. (St. Louis, Mo.).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm can be the distance in Angstroms (Å) from the nucleotide base to the fluorescent moiety. In general, a linker arm is from about 10 Å to about 25 Å. The linker arm may be of the kind described in International Patent Publication No. WO 84/03285. WO 84/03285 also discloses methods for attaching linker arms to a particular nucleotide base, and also for attaching fluorescent moieties to a linker arm.

An acceptor fluorescent moiety, such as an LC Red 640, can be combined with an oligonucleotide that contains an amino linker (e.g., C6-amino phosphoramidites available from ABI (Foster City, Calif.) or Glen Research (Sterling, VA)) to produce, for example, LC Red 640-labeled oligonucleotide. Frequently used linkers to couple a donor fluorescent moiety such as fluorescein to an oligonucleotide include thiourea linkers (FITC-derived, for example, fluorescein-CPG's from Glen Research or ChemGene (Ashland, Mass.)), amide-linkers (fluorescein-NHS-ester-derived, such as CX-fluorescein-CPG from BioGenex (San Ramon, Calif.)), or 3'-amino-CPGs that require coupling of a fluorescein-NHS-ester after oligonucleotide synthesis.

### Detection of Plasmodium Amplified Product (Amplicon)

The present disclosure provides methods for detecting the presence or absence of Malaria parasites (including *Plasmodium*) in a biological or non-biological sample. Methods provided avoid problems of sample contamination, false negatives, and false positives. The methods include performing at least one cycling step that includes amplifying a portion of *Plasmodium* target nucleic acid molecules from a sample using one or more pairs of *Plasmodium* primers, and a FRET detecting step. Multiple cycling steps are performed, preferably in a thermocycler. Methods can be performed using the *Plasmodium* primers and probes to detect the presence of *Plasmodium,* and the detection of *Plasmodium* indicates the presence of *Plasmodium* in the sample.

As described herein, amplification products can be detected using labeled hybridization probes that take advantage of FRET technology. One FRET format utilizes TaqMan^{®} technology to detect the presence or absence of an amplification product, and hence, the presence or absence of Malaria parasites (including *Plasmodium*)*.* TaqMan^{®} technology utilizes one single-stranded hybridization probe labeled with, *e.g.*, one fluorescent moiety or dye (*e.g.*, HEX or FAM) and one quencher (e.g., BHQ-2), which may or may not be fluorescent. When a first fluorescent moiety is excited with light of a suitable wavelength, the absorbed energy is transferred to a second fluorescent moiety or a dark quencher according to the principles of FRET. The second moiety is generally a quencher molecule. During the annealing step of the PCR reaction, the labeled hybridization probe binds to the target DNA (*i.e.*, the amplification product) and is degraded by the 5' to 3' nuclease activity of, *e.g.*, the Taq Polymerase during the subsequent elongation phase. As a result, the fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM^{®} 7700 Sequence Detection System (Applied Biosystems) uses TaqMan^{®} technology, and is suitable for performing the methods described herein for detecting the presence or absence of Malaria parasites (including *Plasmodium*) in the sample.

Molecular beacons in conjunction with FRET can also be used to detect the presence of an amplification product using the real-time PCR methods. Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (*e.g.*, a hairpin). As a result of secondary structure formation within the probe, both fluorescent moieties are in spatial proximity when the probe is in solution. After hybridization to the target nucleic acids (i.e., amplification products), the secondary structure of the probe is disrupted and the fluorescent moieties become separated from one another such that after excitation with light of a suitable wavelength, the emission of the first fluorescent moiety can be detected.

Another common format of FRET technology utilizes two hybridization probes. Each probe can be labeled with a different fluorescent moiety and are generally designed to hybridize in close proximity to each other in a target DNA molecule (e.g., an amplification product). A donor fluorescent moiety, for example, fluorescein, is excited at 470 nm by the light source of the LightCycler^{®} Instrument. During FRET, the fluorescein transfers its energy to an acceptor fluorescent moiety such as LightCycler^{®}-Red 640 (LC Red 640) or LightCycler^{®}-Red 705 (LC Red 705). The acceptor fluorescent moiety then emits light of a longer wavelength, which is detected by the optical detection system of the LightCycler^{®} instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target DNA molecules (*e.g.*, the number of *Plasmodium* genomes). If amplification of *Plasmodium* target nucleic acid occurs and an amplification product is produced, the step of hybridizing results in a detectable signal based upon FRET between the members of the pair of probes.

Generally, the presence of FRET indicates the presence of *Plasmodium* in the sample, and the absence of FRET indicates the absence of *Plasmodium* in the sample. Inadequate specimen collection, transportation delays, inappropriate transportation conditions, or use of certain collection swabs (calcium alginate or aluminum shaft) are all conditions that can affect the success and/or accuracy of a test result, however.

Representative biological samples that can be used in practicing the methods include, but are not limited to whole blood, respiratory specimens, urine, fecal specimens, blood specimens, plasma, dermal swabs, nasal swabs, wound swabs, blood cultures, skin, and soft tissue infections. Collection and storage methods of biological samples are known to those of skill in the art. Biological samples can be processed (*e.g*., by nucleic acid extraction methods and/or kits known in the art) to release *Plasmodium* nucleic acid or in some cases, the biological sample can be contacted directly with the PCR reaction components and the appropriate oligonucleotides. In some instances, the biological sample is whole blood. When whole blood is typically collected, it is often collected in vessels containing anticoagulants, such as heparin, citrate, or EDTA, which enables the whole blood to be stored at suitable temperatures. However, under such conditions, the nucleic acids within the whole blood undergo considerable amount of degradation. Therefore, it may be advantageous to collect the blood in a reagent that will lyse, denature, and stabilize whole blood components, including nucleic acids, such as a nucleic acid-stabilizing solution. In such cases, the nucleic acids can be better preserved and stabilized for subsequent isolation and analysis, such as by nucleic acid test, such as PCR. Such nucleic acid-stabilizing solution are well known in the art, including, but not limited to, cobas PCR media, which contains 4.2 M guanadinium salt (GuHCl) and 50 mM Tris, at a pH of 7.5.

The sample can be collected by any method or device designed to adequately hold and store the sample prior to analysis. Such methods and devices are well known in the art. In the case that the sample is a biological sample, such as whole blood, the method or device may include a blood collection vessel. Such a blood collection vessel is well known in the art, and may include, for example, a blood collection tube. In many cases, it may be advantageous to use a blood collection tube wherein the blood collection vessel is under pressure in the space intended for sample uptake, such as a blood vessel with an evacuated chamber, such as a vacutainer blood collection tube. Such blood collection tubes with an evacuated chamber, such as a vacutainer blood collection tube are well known in the art. It may further be advantageous to collect the blood in a blood collection vessel, with or without an evacuated chamber, that contains within it, a solution that will lyse, denature, and stabilize whole blood components, including nucleic acids, such as a nucleic acid-stabilizing solution, such that the whole blood being drawn immediately contacts the nucleic acid-stabilizing solution in the blood collection vessel.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the *Plasmodium* probes from the *Plasmodium* amplification products can confirm the presence or absence of *Plasmodium* in the sample.

Within each thermocycler run, control samples can be cycled as well. Positive control samples can amplify target nucleic acid control template (other than described amplification products of target genes) using, for example, control primers and control probes. Positive control samples can also amplify, for example, a plasmid construct containing the target nucleic acid molecules. Such a plasmid control can be amplified internally (*e.g*., within the sample) or in a separate sample run side-by-side with the patients' samples using the same primers and probe as used for detection of the intended target. Such controls are indicators of the success or failure of the amplification, hybridization, and/or FRET reaction. Each thermocycler run can also include a negative control that, for example, lacks target template DNA. Negative control can measure contamination. This ensures that the system and reagents would not give rise to a false positive signal. Therefore, control reactions can readily determine, for example, the ability of primers to anneal with sequence-specificity and to initiate elongation, as well as the ability of probes to hybridize with sequence-specificity and for FRET to occur.

In an embodiment, the methods include steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next.

Conventional PCR methods in conjunction with FRET technology can be used to practice the methods. In one embodiment, a LightCycler^{®} instrument is used. The following patent applications describe real-time PCR as used in the LightCycler^{®} technology: International Patent Publication Nos. WO 97/46707, WO 97/46714, and WO 97/46712.

The LightCycler^{®} can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (e.g., SYBR^{®} Green or SYBR^{®} Gold (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

One of skill in the art would appreciate that other nucleic acid- or signal-amplification methods may also be employed. Examples of such methods include, without limitation, branched DNA signal amplification, loop-mediated isothermal amplification (LAMP), nucleic acid sequence-based amplification (NASBA), self-sustained sequence replication (3SR), strand displacement amplification (SDA), or smart amplification process version 2 (SMAP 2).

It is understood that the embodiments of the present disclosure are not limited by the configuration of one or more commercially available instruments.

### Articles of Manufacture/Kits

The present disclosure further provides for articles of manufacture or kits to detect Malaria parasites (including *Plasmodium*)*.* An article of manufacture can include primers and probes used to detect the *Plasmodium* gene target, together with suitable packaging materials. Representative primers and probes for detection of *Plasmodium* are capable of hybridizing to *Plasmodium* target nucleic acid molecules. In addition, the kits may also include suitably packaged reagents and materials needed for DNA immobilization, hybridization, and detection, such solid supports, buffers, enzymes, and DNA standards. Methods of designing primers and probes are disclosed herein, and representative examples of primers and probes that amplify and hybridize to *Plasmodium* target nucleic acid molecules are provided.

Articles of manufacture can also include one or more fluorescent moieties for labeling the probes or, alternatively, the probes supplied with the kit can be labeled. For example, an article of manufacture may include a donor and/or an acceptor fluorescent moiety for labeling the *Plasmodium* probes. Examples of suitable FRET donor fluorescent moieties and corresponding acceptor fluorescent moieties are provided above.

Articles of manufacture can also contain a package insert or package label having instructions thereon for using the *Plasmodium* primers and probes to detect *Plasmodium* in a sample. Articles of manufacture may additionally include reagents for carrying out the methods disclosed herein (*e.g.*, buffers, polymerase enzymes, co-factors, or agents to prevent contamination). Such reagents may be specific for one of the commercially available instruments described herein.

The present disclosure also provides for a set of primers and one or more detectable probes for the detection of Malaria parasites (including *Plasmodium*) in a sample.

Embodiments of the present disclosure will be further described in the following examples, which do not limit the scope of the invention described in the claims.

### EXAMPLES

The following examples and figures are provided to aid the understanding of the subject matter, the true scope of which is set forth in the appended claims.

The test was a fully automated sample preparation (nucleic acid extraction and purification) followed by PCR amplification and detection. The system used was the cobas^{®} 6800/8800 System, which consisted of a sample supply module, the transfer module, the processing module, and the analytic module.

Selective amplification of target nucleic acid was achieved by the use of specific forward and reverse primers, which were selected from highly conserved regions of the target nucleic acid. A thermostable DNA polymerase enzyme was used for both reverse-transcription and amplification. The master mix included deoxyuridine triphosphate (dUTP), instead of deoxythimidine triphosphate (dTTP), which is incorporated into the newly synthesized DNA (amplified product or amplicon). Any contaminating amplicons from previous PCR runs were destroyed by the AmpErase enzyme (uracil-N-glycosylase), which was included in the PCR mix, when heated in the first thermal cycling step. Newly formed amplicons were not destroyed, however, since the AmpErase enzyme was inactivated once exposed to temperatures above 55°C.

The cobas^{®} *Plasmodium* master mix contained detection probes, which were specific for *Plasmodium* and control nucleic acids. The specific *Plasmodium* and control detection probes were each labeled with one of two unique fluorescent dyes, which act as a reporter. Each probe also had a second dye, which acted as a quencher. The reporter dye was measured at a defined wavelength, thus permitting detection and discrimination of the amplified *Plasmodium* target and the control. The fluorescent signal of the intact probes was suppressed by the quencher dye. During the PCR amplification step, hybridization of the probes to the specific single-stranded DNA template resulted in cleavage by the 5' to 3' nuclease activity of the DNA polymerase resulting in separation of the reporter and quencher dyes, and the generation of fluorescent signal. With each PCR cycle, increasing amounts of cleaved probes were generated and the cumulative signal of the reporter dye was concomitantly increased. Because the two specific reporter dyes are measured at defined wavelengths, simultaneous detection and discrimination of the amplified *Plasmodium* target and the control was possible.

The targeted region of the *Plasmodium* genome was the 18S ribosomal RNA and the 28S ribosomal RNA. The disclosed Malaria parasite assay is designed to be a pan-Malaria assay, which is able to detect the following *Plasmodium* species: *P. falciparum*, *P. vivax*, *P. malariae*, *P. ovale*, and *P. knowlesi.* The assay excludes target sequences that share homology with closely related species (e.g., parasites and bacteria) and humans, as well as environmental DNA that may be found in the various assay reagents. The Malaria assay is designed to detect *Plasmodium* species in samples, such as biological samples, such as whole blood. The disclosed Malaria assay detects *Plasmodium* species in about 1.1 ml of whole blood. In some circumstances, the whole blood sample is in a tube/vessel, such as an evacuated tube/vessel that also contains within it a reagent/solution that will lyse, denature, and stabilize whole blood components, including nucleic acids, such as a nucleic acid-stabilizing solution, such that the whole blood being drawn immediately contacts the nucleic acid-stabilizing solution in the blood collection vessel. The disclosed primer pairs having nucleic acid sequences of SEQ ID NOs: 1 and 2, and the disclosed probes having a nucleic acid sequence of SEQ ID NOs:3 and/or 4, detect and/or amplify an 18S ribosomal RNA target. The disclosed primer pairs having nucleic acid sequences of SEQ ID NOs: 13 and 14, and the disclosed probes having a nucleic acid sequence of SEQ ID NO:15, detect and/or amplify a 28S ribosomal RNA target.

Thus, the assay for detection of Malaria parasites is a qPCR-based assay was developed to detect the *Plasmodium* species: *P. falciparum*, *P. vivax*, *P. ovale*, *P. knowlesi*, and *P. malariae*, which cause malaria, in blood samples. This qPCR assay was designed for the cobas 6800/8800 platform. Although the Unites States is considered a non-endemic country, global travel may lead to an increase in the cases of imported malaria, thereby, affecting the blood supply. There are two strategies regarding malaria that are employed to maintain safety of blood supplies: (1) Selective Testing, and (2) Donor Deferral. Currently, the United States has a donor deferral policy for blood donations, where donors are deferred based on questionnaire answers regarding their history of malaria infection, prior residency of an endemic country, and travel to an endemic country. Donor deferrals negatively impact the blood supply by excluding donations based on questionnaire answers, regardless of malaria status. On the other hand, selective testing reduces the impact on blood supply inventory as compared to donor deferral. Selective testing of blood donations is also based on questionnaire; however, the selective testing policy allows for shorter deferral times and reinstatement of donors depending on the donor's malaria status. In countries that have the selective testing policy, testing is done via enzyme immunoassays. There are, at present, no IVD nucleic acid tests that are currently available for screening blood donations. In comparison to enzyme immunoassays, nucleic acid tests are more sensitivity at detecting malaria parasites in blood. The assay's design strategy for the qPCR based test was to target genes that are present in multiple copies within the cell. This test targets 28S and 18S ribosomal RNA. Detection of multiple copy DNA and RNA targets in parallel allows for increased sensitivity. This qPCR assay can be used by hospitals and blood centers to screen for malaria in collected blood donations.

### Example 1: Design of 18S rRNA Target Assay

The 18S rRNA target nucleic acid assay was tested using *P. falciparum* from *P. falciparum* cultures from ATCC (Catalog No. 30932). Four different regions based on publications (Seilie, et al., "Beyond Blood Smears: Qualification of Plasmodium 18S rRNA as a Biomarker for Controlled Human Malaria Infections," The American Journal of Tropical Medicine and Hygiene 100(6):1466-1476 (2019); Rougemont, et al., "Detection of Four Plasmodium Species in Blood from Humans by 18S rRNA Gene Subunit-Based and Species-Specific Real-Time PCR Assays," Journal of Clinical Microbiology 42(12):5636-5643 (2004); Kamau, et al., "Development of a Highly Sensitive Genus-Specific Quantitative Reverse Transcriptase Real-Time PCR Assay for Detection and Quantitation of Plasmodium by Amplifying RNA and DNA of the 18S rRNA Genes," Journal of Clinical Microbiology 49(8):2946-2953 (2011); Waggoner, et al., "Multiplex Nucleic Acid Amplification Test for Diagnosis of Dengue Fever, Malaria, and Leptospirosis," Journal of Clinical Microbiology 52(6):2011-2018 (2014)) of the 18S rRNA were considered, as shown in **FIG. 1****.** Reagents used include cobas^{®} 6800/8800 generic PCR Master Mix, with the profile and conditions for use with the cobas^{®} 6800/8800, and using TaqMan^{®} amplification and detection technology. The final concentration of oligonucleotides in the master mix was 0.3 µM for primers and 0.1 µM for probes. The cobas^{®} 6800/8800 PCR Profile employed throughout in these Examples, is depicted in Table 3, below:

**Table 3**

| cobas^{®} 6800/8800 PCR Profile | | | | |
|---|---|---|---|---|
| Step | Cycles | Target (°C) | Hold time (hh:mm:ss) | Ramp |
| Pre-PCR | 1 | 50 | 00:02:00 | 4.4 |
| | | 94 | 00:00:05 | 4.4 |
| | | 55 | 00:02:00 | 2.2 |
| | | 60 | 00:06:00 | 4.4 |
| | | 65 | 00:04:00 | 4.4 |
| 1. Meas | 5 | 95 | 00:00:05 | 4.4 |
| | | 55 | 00:00:30 | 2.2 |
| 2. Meas | 45 | 91 | 00:00:05 | 4.4 |
| | | 58 | 00:00:25 | 2.2 |
| Post | 1 | 40 | 00:02:00 | 2.2 |

Each of the four candidates were evaluated with *P. falciparum* ATCC culture eluates in a whole blood eluate background. *Babesia* cross-reactivity testing was also conducted simultaneously (data not shown). Of the four candidate regions evaluated, the oligonucleotides targeting the region between 1,290,600 and 1,290,800 bases mapping to 18S rRNA on chromosome 5 of *P*. *falciparum* 3D7 showed the highest RFI (data not shown), and was selected for further evaluation. Several additional re-designed candidate oligonucleotides targeting the region between 1,290,600 and 1,290,800 bases mapping to 18S rRNA on chromosome 5 of *P*. *falciparum* 3D7 were tested. These candidate oligonucleotides were mapped against *Plasmodium* species as well as *Babesia,* as shown in **FIG. 2****.** Different combinations of the re-designed oligonucleotides were tested with *P*. *falciparum* ATCC cultures in a whole blood eluate background, again. The results are shown in **FIG. 3****.** As seen in **FIG. 3****,** some combinations showed high cross reactivity with *Babesia* (as indicated by the asterisks). The primer set comprising a forward primer, designated 1073F, and a reverse primer, designated 1262R showed the highest RFI, while exhibiting no cross-reactivity with *Babesia.*

Thus, these studies demonstrate numerous candidate primers/probes design for the 18S rRNA target were effective at detecting and amplifying the 18S rRNA target from *Plasmodium* samples in a whole blood eluate setting.

### Example 2: Design of 28S rRNA Target Assay

The 28S rRNA target nucleic acid assay was tested using *P. falciparum* from *P. falciparum* cultures from ATCC (Catalog No. 30932). As in Example 1, the reagents used include cobas^{®} 6800/8800 generic PCR Master Mix, with the profile and conditions for use with the cobas^{®} 6800/8800, and using TaqMan^{®} amplification and detection technology. The final concentration of oligonucleotides in the master mix was 0.3 µM for primers and 0.1 µM for probes. The cobas^{®} 6800/8800 PCR Profile employed is depicted in Table 3, above.

Combinations of three different forward primer candidates, two different reverse primer candidates, and one probe were tested, targeting the region of the 28S rRNA from chromosome 5, as shown in **FIG. 4****.** Each of the candidates were evaluated with *P. falciparum* and *P. vivax* (*P. vivax* data not shown) ATCC culture eluates in a whole blood eluate background. Babesia cross-reactivity testing was also conducted simultaneously (data not shown). These studies showed that all combinations of primers all showed ability to detect and amplify *F. falciparum* and *F*. *vivax* (*P. vivax* data not shown), as can be seen in **FIG. 5****.** However, two of the primer combinations exhibited cross reactivity with Babesia (as indicated by the asterisks in **FIG. 5****),** and were not considered further.

Thus, these studies demonstrate numerous candidate primers/probes design for the 28S rRNA target were effective at detecting and amplifying the 28S rRNA target from *Plasmodium* samples in a whole blood eluate setting.

### Example 3: Modifications of 18S and 28S rRNA Candidate Primers/Probes for Improved Performance

The list of candidate primers/probes for the 18S and 28S, as discussed on Examples 1 and 2, was then subsequently subject to modifications for potentially further improve and enhance performance. To that end, the primers were modified to assess if performance could be improved. Modified primers for the 18S and 28S rRNA targets were tested in a singleplex assay using *P*. *falciparum* from *P. falciparum* cultures from ATCC (Catalog No. 30932). As in Examples 1 and 2, the reagents used include cobas^{®} 6800/8800 generic PCR Master Mix, with the profile and conditions for use with the cobas^{®} 6800/8800, and using TagMan^{®} amplification and detection technology. The final concentration of oligonucleotides in the master mix was 0.3 µM for primers and 0.1 µM for probes. The cobas^{®} 6800/8800 PCR Profile employed is depicted in Table 3, above. Results are depicted in **FIG. 6A****.** All combinations of primers/probes showed an ability to detect and amplify 18S or 28S rRNA *P. falciparum* target nucleic acid from cultures in a whole blood background. Therefore, the single target assays exhibiting the best RFIs (designated by the asterisks in **FIG. 6A****)** were selected for further testing in a dual target assay configuration. The dual target assay configuration is depicted in **FIG. 6B****,** which shows the 18S and 28S rRNA target primers/probes in the FAM channel, and the internal control in the Cy5.5 channel. The ideal 18S rRNA target primers/probes correspond to nucleic acid sequences of SEQ ID NOs:1-3, and the ideal 28S rRNA target primer/probes correspond to nucleic acid sequences of SEQ ID NOs:13-15. It is noted that the probe used for detecting the 18S rRNA target (*i.e.*, having the sequence of SEQ ID NO:3) originated from a probe for hybridizing to the 18S rRNA target from a 2019 publication (Seilie, et al., "Beyond Blood Smears: Qualification of Plasmodium 18S rRNA as a Biomarker for Controlled Human Malaria Infections," Am. J. Trop. Med. Hyg. 100(6):1466-1476 (2019)). The sequences of the 18S/28S rRNA dual-target Plasmodium assay is shown in Table 4, below.

**Table 4: Sequences of the 18S/28S rRNA dual-target Plasmodium assay**

| **Type** | **Oligo ID** | **SEQ ID.** | **Sequence** |
|---|---|---|---|
| **18S rRNA Target Oligonucleotides** | | | |
| F. Primer | PanDDT1073FMO D | 1 | TGCGAAAGCATTTGCCTAAAATACTT<t_BB_dC> |
| R. Primer | PanDDT1262R | 2 | CAGAACCCAAAGACTTTGATTTCTCAT |
| Probe | PanDDTprobe | 3 | <FAM_Thr>ACCGT<BHQ_2>CGTAATCTTAACCATAAACT ATGCCGACTAG<Spc_C3> |
| **28S rRNA Target Oligonucleotides** | | | |
| F. Primer | 28S_1179_1200Fm od | 13 | GACCCGAGAGGCTTTGAACTA<t_BB_dA> |
| R. Primer | 28S_1346_1318R | 14 | GATCCTGAGAGATCTTTCGAAAATAACCA |
| Probe | 28S_1284_1316P1 | 15 | <FAM_Thr>TAGGG<BHQ_2>GCGAAAGA<pdC><pdU>AA<p dU><pdC>GAAAAG<pdC><pdC><pdU>ATTAG<Spc_C3> |

Thus, these studies demonstrate that the modified primers/probes for the 18S rRNA target (corresponding to SEQ ID NOs:1-3) and the modified primers/probes for the 28S rRNA target (corresponding to SEQ ID NOs:13-15) are effective at detecting and amplifying the 18S and 28S rRNA target nucleic acids, respectively, from *P. falciparum* cultures in a whole blood setting.

### Example 4: Inclusivity Analysis

The ribosomal RNA (rRNA) targets, 18S and 28S rRNA, were selected because they are present in multiple copies. Across different species of *Plasmodium,* rRNA sequences on different chromosomes are conserved, but not identical, and rRNA expression is dependent on developmental stage. Chromosome rRNA expression is not well characterized for less predominant species.

An *in silico* inclusivity analysis was conducted, using a Bioinformatics PLR tool. These results of this analysis demonstrated that the 18S rRNA target has more deposited sequences than 28S rRNA targets, for all *Plasmodium* sequences (data not shown). Further, the majority of 18S and 28S targets are predicted to be covered by the assay, which includes both A- and S-types. Indeed, it was determined that, with 4-8 rRNA gene copies per genome, if one copy is predicted to fail, other copies will provide coverage (data not shown). Thus, as can be seen in **FIG. 7A** and **7B****,** which shows alignment of the candidate dual target assay primers/probes with targets, the 18S/28S rRNA dual-target assay provides added inclusivity.

In order to confirm species inclusivity, minigenes containing full length 18S rRNA from each species of *Plasmodium* (*P. falciparum*, *P. vivax*, *P. malariae*, *P. ovale*, and *P. knowlesi*) were tested by a confirmatory assay *(i.e.,* the instant 18S/28S rRNA dual-target assay with primers/probes with sequences SEQ ID NOs:1-3, for 18S rRNA targets, and primers/probes with sequences SEQ ID NOs:13-15, for 28S rRNA targets), as compared to an existing 18S-only dual-target assay *(i.e.,* a dual-target assay with both targets in the 18S rRNA region). **FIG. 8** shows the alignment of these two different assays. The results are shown in **FIG. 9A****,** which shows data confirming that the instant 18S/28S rRNA dual-target assay (with primers/probes with sequences SEQ ID NOs:1-3, for 18S rRNA targets, and primers/probes with sequences SEQ ID NOs:13-15, for 28S rRNA targets) is able to detect all *Plasmodium* species, including *P. falciparum*, *P. vivax*, *P. malariae*, *P. ovale*, and *P. knowlesi.* In particular, **FIG. 9B** shows the results for *P. falciparum*; **FIG. 9C** shows the results for *P. vivax*; **FIG. 9D** shows the results for *P. malariae*; **FIG. 9E** shows the results for *P. ovale*; and **FIG. 9F** shows the results for *P. knowlesi.*

Taken together, these studies demonstrate, unequivocally, that the instant 18S/28S rRNA dual-target assay (with primers/probes with sequences SEQ ID NOs:1-3, for 18S rRNA targets, and primers/probes with sequences SEQ ID NOs:13-15, for 28S rRNA targets) is able to detect all *Plasmodium* species.

### Example 5: Analysis of Specificity of 18S/28S rRNA Dual-Target Assay for Plasmodium Species Only

Having demonstrated that the instant 18S/28S rRNA dual-target assay (with primers/probes with sequences SEQ ID NOs:1-3, for 18S rRNA targets, and primers/probes with sequences SEQ ID NOs:13-15, for 28S rRNA targets) is able to detect all *Plasmodium* species, it was critical to confirm that the instant 18S/28S rRNA dual-target assay did not exhibit cross reactivity to other non-*Plasmodium* targets. To that end, a panel of non-*Plasmodium* samples (including *Babesia, Haemophilus influenza, Candida albicans, Cutibacterium acnes, Klebsiella pneumoniae, Stephaylococcus aureus*, *Staphylococcus epidermidis*, HIV, Hepatitis B Virus (HBV), Hepatitis C Virus (HCV), and West Nile Virus) was tested with the instant 18S/28S rRNA dual-target assay.

These studies showed that the 18S/28S rRNA dual-target assay is not reactive to any of the non-*Plasmodium* samples tested, as shown in **FIG. 10****.**

Thus, these studies demonstrate that the 18S/28S rRNA dual-target assay (with primers/probes with sequences SEQ ID NOs:1-3, for 18S rRNA targets, and primers/probes with sequences SEQ ID NOs:13-15, for 28S rRNA targets) is specific *for Plasmodium* species, and does not cross react with any of *Babesia*, *Haemophilus influenza*, *Candida albicans*, *Cutibacterium acnes*, *Klebsiella pneumoniae*, *Stephaylococcus aureus*, *Staphylococcus epidermidis*, HIV, Hepatitis B Virus (HBV), Hepatitis C Virus (HCV), and West Nile Virus.

### Example 6: Analysis of Sensitivity of 18S/28S rRNA Dual-Target Plasmodium Assay

Having demonstrated that the instant 18S/28S rRNA dual-target assay (with primers/probes with sequences SEQ ID NOs:1-3, for 18S rRNA targets, and primers/probes with sequences SEQ ID NOs:13-15, for 28S rRNA targets) is able to specifically detect all *Plasmodium* species, it was critical to analyze the sensitivity of the 18S/28S rRNA dual-target assay. To that end, the 18S/28S rRNA dual-target assay was tested using nucleic acids from *P. falciparum* and *P. vivax* cultures from ATCC (Catalog Nos. 30932 and 30139) were extracted on the cobas 6800/8800, and then blended with whole blood eluates. These samples were then subject to the 18S/28S rRNA dual-target assay. As in Examples 1-3, the reagents used include cobas^{®} 6800/8800 generic PCR Master Mix, with the profile and conditions for use with the cobas^{®} 6800/8800, and using TaqMan^{®} amplification and detection technology. The final concentration of oligonucleotides in the master mix was 0.3 µM for primers and 0.1 µM for probes. The cobas^{®} 6800/8800 PCR Profile employed is depicted in Table 3, above.

The results are depicted in **FIGs. 11A****,** **11B****,** and **11C****.** **FIG. 11A** depicts the data, showing that the 18S/28S rRNA dual-target assay is sensitive enough to detect 0.000224 parasites/ml for F. *falciparum,* and 0.0000536 parasites/ml for F. *vivax.* **FIG. 11B** and **FIG. 11C** show the PCR growth curves for *P. falciparum* and *P. vivax,* respectively.

Thus, these studies demonstrate that the 18S/28S rRNA dual-target assay (with primers/probes with sequences SEQ ID NOs:1-3, for 18S rRNA targets, and primers/probes with sequences SEQ ID NOs:13-15, for 28S rRNA targets) is a very sensitive assay, capable of detecting even very low amounts/levels of *Plasmodium* species.

### Example 7: Full Process with Plasmodium-Positive Clinical Specimen with 18S/28S rRNA Dual-Target Plasmodium Assay

In order to assess if the 18S/28S rRNA dual-target *Plasmodium* assay was effective on actual clinical samples, a full process study was conducted. In this study, clinical samples known to be positive for Plasmodium (as confirmed by microscopy) were employed, and subject to the 18S/28S rRNA dual-target *Plasmodium* assay. These Plasmodium-positive clinical specimens were serially diluted in negative whole blood. As in Examples 1-3 and 6, the reagents used include cobas^{®} 6800/8800 generic PCR Master Mix, with the profile and conditions for use with the cobas^{®} 6800/8800, and using TaqMan^{®} amplification and detection technology. The final concentration of oligonucleotides in the master mix was 0.3 µM for primers and 0.1 µM for probes. The cobas^{®} 6800/8800 PCR Profile employed is depicted in Table 3, above

The results are depicted in **FIG. 12****,** showing that the 18S/28S rRNA dual-target *Plasmodium* assay is able to detect and amplify *P. falciparum* and *P*. *vivax* nucleic acids from positive clinical samples. Thus, these studies demonstrate that the 18S/28S rRNA dual-target assay (with primers/probes with sequences SEQ ID NOs: 1-3, for 18S rRNA targets, and primers/probes with sequences SEQ ID NOs: 13-15, for 28S rRNA targets) is able to detect *Plasmodium* from clinical samples.

Thus, taken together, these Examples and results demonstrate that the oligonucleotide set of SEQ ID NOs: 1-39, particularly primers/probes comprising SEQ ID NOs: 1-3 and primers/probes with sequences comprising SEQ ID NOs:13-15, specifically and efficiently amplify and detect Malaria parasites, including *Plasmodium* (which includes *P. falciparum*, *P. vivax*, *P. ovale*, *P. knowlesi*, and *P. malariae*), in whole blood. Furthermore, these Examples and results demonstrate that the oligonucleotide set of SEQ ID NOs:1-39, particularly primers/probes comprising SEQ ID NOs: 1-3 and primers/probes with sequences comprising SEQ ID NOs:13-15, can be employed in a multiplex assay, such as a dual-target assay, to specifically and sensitively detect and amplify all Plasmodium species in clinical samples, such as whole blood.

## Claims

1. A method for detecting one or more Malaria parasite species belonging to the genus *Plasmodium* in a sample, the method comprising:
(a) performing an amplification step comprising contacting the sample with one or more sets of oligonucleotide primers to produce an amplification product, if a target nucleic acid of the one or more Malaria parasite species is present in the sample;
(b) performing a hybridization step comprising contacting one or more oligonucleotide probes with the amplification product, if a target nucleic acid of the one or more Malaria parasite species is present in the sample; and
(c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and
wherein the one or more sets of oligonucleotide primers and the one or more oligonucleotide probes comprise:
(1) a set of oligonucleotide primers comprising oligonucleotide primers comprising the nucleic acid sequences of SEQ ID NOs:1 and 2; and an oligonucleotide probe comprising the nucleic acid sequence of SEQ ID NO:3, or a complement thereof; and/or
(2) a set of oligonucleotide primers comprising oligonucleotide primers comprising the nucleic acid sequence of SEQ ID NOs:13 and 14; and an oligonucleotide probe comprising the nucleic acid sequence of SEQ ID NO:15, or a complement thereof.

2. A method for detecting a first target nucleic acid and/or a second target nucleic acid of one or more Malaria parasite species belonging to the genus *Plasmodium* in a sample, the method comprising:
(a) performing an amplification step comprising contacting the sample with one or more sets of oligonucleotide primers to produce an amplification product, if the first target nucleic acid and/or the second target nucleic acid of the one or more Malaria parasite species is present in the sample;
(b) performing a hybridization step comprising contacting one or more oligonucleotide probes with the amplification product, if the first target nucleic acid and/or the second target nucleic acid of the one or more Malaria parasite species is present in the sample; and
(c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and
wherein the one or more sets of oligonucleotide primers and the one or more oligonucleotide probes comprise:
(1) a set of oligonucleotide primers and an oligonucleotide probe for the first target nucleic acid of the one or more Malaria parasite species, wherein the set of oligonucleotide primers comprises oligonucleotide primers comprising the nucleic acid sequences of SEQ ID NOs:1 and 2; and wherein the oligonucleotide probe comprises the nucleic acid sequence of SEQ ID NO:3, or a complement thereof; and
(2) a set of oligonucleotide primers and an oligonucleotide probe for the second target nucleic acid of the one or more Malaria parasite species, wherein the set of oligonucleotide primers comprises oligonucleotide primers comprising the nucleic acid sequences of SEQ ID NOs: 13 and 14; and wherein the oligonucleotide probe comprises the nucleic acid sequence of SEQ ID NO:15, or a complement thereof.

3. A method for detecting one or more Malaria parasite species belonging to the genus *Plasmodium* in a sample, the method comprising:
(a) performing an amplification step comprising contacting the sample with one or more sets of oligonucleotide primers to produce an amplification product, if a target nucleic acid of the one or more Malaria parasite species is present in the sample;
(b) performing a hybridization step comprising contacting one or more oligonucleotide probes with the amplification product, if the target nucleic acid of the one or more Malaria parasite species is present in the sample; and
(c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and
wherein the one or more set of oligonucleotide primers and the one or more oligonucleotide probes comprise a set of oligonucleotide primers comprising oligonucleotide primers comprising the nucleic acid sequences of SEQ ID NOs:1 and 2; and an oligonucleotide probe comprising the nucleic acid sequence of SEQ ID NO:3, or a complement thereof.

4. A method for detecting one or more Malaria parasite species belonging to the genus *Plasmodium* in a sample, the method comprising:
(a) performing an amplification step comprising contacting the sample with one or more sets of oligonucleotide primers to produce an amplification product, if a target nucleic acid of the one or more Malaria parasite species is present in the sample;
(b) performing a hybridization step comprising contacting one or more oligonucleotide probes with the amplification product, if the target nucleic acid of the one or more Malaria parasite species is present in the sample; and
(c) detecting the presence or absence of the amplification product, wherein the presence of the amplification product is indicative of the presence of the one or more Malaria parasite species in the sample, and wherein the absence of the amplification product is indicative of the absence of the one or more Malaria parasite species in the sample; and
wherein the one or more sets of oligonucleotide primers and the one or more oligonucleotide probes comprise a set of oligonucleotide primers comprising oligonucleotide primers comprising the nucleic acid sequences of SEQ ID NOs: 13 and 14, or complements thereof; and an oligonucleotide probe comprising the nucleic acid sequence of SEQ ID NO:15, or a complement thereof.

5. The method of any one of claims 1 to 4, wherein the one or more Malaria parasite species that belongs to the genus *Plasmodium* is *P. falciparum, P. vivax, P. ovale, P. malariae*, and/or *P. knowlesi.*

6. The method of any one of claims 1 to 5, wherein the sample is a biological sample and is selected from whole blood, respiratory specimens, urine, fecal specimens, blood specimens, plasma, dermal swabs, nasal swabs, wound swabs, blood cultures, skin, or soft tissue infections.

7. The method of any one of claims 1 to 6, wherein at least one of the one or more oligonucleotide probes is labeled with a donor fluorescent moiety and a corresponding acceptor moiety.

8. The method of claim 7, wherein step (c) further comprises detecting the presence or absence of fluorescent resonance energy transfer (FRET) between the donor fluorescent moiety and the acceptor moiety of the one or more oligonucleotide probes, wherein the presence or absence of fluorescence is indicative of the presence of absence of the one or more Malaria parasite species in the sample.

9. A kit for detecting one or more Malaria parasite species belonging to the genus *Plasmodium* that may be present in a sample, the kit comprising amplification reagents comprising at least one of a DNA polymerase and nucleotide monomers, and one or more sets of oligonucleotide primers and one or more oligonucleotide probes, wherein the one or more sets of oligonucleotide primers and the one or more oligonucleotide probes comprise:
(1) a set of oligonucleotide primers comprising oligonucleotide primers comprising the nucleic acid sequences of SEQ ID NOs:1 and 2; and an oligonucleotide probe comprising the nucleic acid sequence of SEQ ID NO:3, or a complement thereof; and/or
(2) a set of oligonucleotide primers comprising oligonucleotide primers comprising the nucleic acid sequences of SEQ ID NOs:13 and 14; and an oligonucleotide probe comprising the nucleic acid sequence of SEQ ID NO:15, or a complement thereof.

10. A kit for detecting a first target nucleic acid and/or a second target nucleic acid of one or more Malaria parasite species belonging to the genus *Plasmodium* that may be present in a sample, the kit comprising amplification reagents comprising at least one of a DNA polymerase and nucleotide monomers, and one or more sets of oligonucleotide primers and one or more oligonucleotide probes, wherein the one or more sets of oligonucleotide primers and the one or more oligonucleotide probes comprise:
(1) a set of oligonucleotide primers and an oligonucleotide probe for the first target nucleic acid of the one or more Malaria parasite species, wherein the set of oligonucleotide primers comprises oligonucleotide primers comprising the nucleic acid sequences of SEQ ID NOs:1 and 2; and wherein the oligonucleotide probe comprises the nucleic acid sequence of SEQ ID NO:3, or a complement thereof; and
(2) a set of oligonucleotide primers and an oligonucleotide probe for the second target nucleic acid of the one or more Malaria parasite species, wherein the set of oligonucleotide primers comprises oligonucleotide primers comprising the nucleic acid sequences of SEQ ID NOs:13 and 14; and wherein the oligonucleotide probe comprises the nucleic acid sequence of SEQ ID NO:15, or a complement thereof.

11. A kit for detecting one or more Malaria parasite species belonging to the genus *Plasmodium* that may be present in a sample, the kit comprising amplification reagents comprising at least one of a DNA polymerase and nucleotide monomers, and one or more sets of oligonucleotide primers and one or more oligonucleotide probes,
wherein the one or more sets of oligonucleotide primers and the one or more probes comprise: a set of oligonucleotide primers comprising oligonucleotide primers comprising the nucleic acid sequences of SEQ ID NOs:1 and 2; and an oligonucleotide probe comprising the nucleic acid sequence of SEQ ID NO:3, or a complement thereof.

12. A kit for detecting one or more Malaria parasite species belonging to the genus *Plasmodium* that may be present in a sample, the kit comprising amplification reagents comprising at least one of a DNA polymerase and nucleotide monomers, and one or more sets of oligonucleotide primers and one or more oligonucleotide probes,
wherein the one or more sets of oligonucleotide primers and the one or more oligonucleotide probes comprise: a set of oligonucleotide primers comprising oligonucleotide primers comprising the nucleic acid sequences of SEQ ID NOs: 13 and 14; and an oligonucleotide probe comprising the nucleic acid sequence of SEQ ID NO:15, or a complement thereof.

13. The kit of any one of claims 9 to 12, wherein the one or more Malaria parasite species that belongs to the genus *Plasmodium* is *P. falciparum, P. vivax, P. ovale, P. malariae*, and/or *P*. *knowlesi.*

14. The kit of any one of claims 9 to 13, wherein the sample is a biological sample and is selected from whole blood, respiratory specimens, urine, fecal specimens, blood specimens, plasma, dermal swabs, nasal swabs, wound swabs, blood cultures, skin, or soft tissue infections.

15. The kit of any one of claims 9 to 14, wherein at least one of the one or more oligonucleotide probes is labeled with a donor fluorescent moiety and a corresponding acceptor moiety.

## Patentansprüche

1. Verfahren zum Nachweis einer oder mehrerer Malaria-Parasitenarten, die zur Gattung *Plasmodium* gehören, in einer Probe, wobei das Verfahren Folgendes umfasst:
(a) Durchführen eines Amplifikationsschritts, umfassend das Inkontaktbringen der Probe mit einem oder mehreren Sätzen von Oligonukleotidprimern, um ein Amplifikationsprodukt zu erzeugen, wenn eine Zielnukleinsäure der einen oder der mehreren Malaria-Parasitenarten in der Probe vorhanden ist;
(b) Durchführen eines Hybridisierungsschritts, umfassend das Inkontaktbringen einer oder mehrerer Oligonukleotidsonden mit dem Amplifikationsprodukt, wenn eine Zielnukleinsäure der einen oder der mehreren Malaria-Parasitenarten in der Probe vorhanden ist; und
(c) Nachweisen des Vorhandenseins oder Nichtvorhandenseins des Amplifikationsprodukts, wobei das Vorhandensein des Amplifikationsprodukts das Vorhandensein der einen oder der mehreren Malaria-Parasitenarten in der Probe anzeigt und wobei das Nichtvorhandensein des Amplifikationsprodukts das Nichtvorhandensein der einen oder der mehreren Malaria-Parasitenarten in der Probe anzeigt; und
wobei der eine oder die mehreren Sätze von Oligonukleotidprimern und die eine oder die mehreren Oligonukleotidsonden Folgendes umfassen:
(1) einen Satz von Oligonukleotidprimern, umfassend Oligonukleotidprimer, umfassend die Nukleinsäuresequenzen von SEQ ID NO: 1 und 2; und eine Oligonukleotidsonde, umfassend die Nukleinsäuresequenz von SEQ ID NO: 3 oder ein Komplement davon; und/oder
(2) einen Satz von Oligonukleotidprimern, umfassend Oligonukleotidprimer, umfassend die Nukleinsäuresequenz von SEQ ID NO: 13 und 14; und eine Oligonukleotidsonde, umfassend die Nukleinsäuresequenz von SEQ ID NO: 15 oder ein Komplement davon.

2. Verfahren zum Nachweis einer ersten Zielnukleinsäure und/oder einer zweiten Zielnukleinsäure einer oder mehrerer Malaria-Parasitenarten, die zur Gattung *Plasmodium* gehören, in einer Probe, wobei das Verfahren Folgendes umfasst:
(a) Durchführen eines Amplifikationsschritts, umfassend das Inkontaktbringen der Probe mit einem oder mehreren Sätzen von Oligonukleotidprimern, um ein Amplifikationsprodukt zu erzeugen, wenn die erste Zielnukleinsäure und/oder die zweite Zielnukleinsäure der einen oder der mehreren Malaria-Parasitenarten in der Probe vorhanden ist;
(b) Durchführen eines Hybridisierungsschritts, umfassend das Inkontaktbringen einer oder mehrerer Oligonukleotidsonden mit dem Amplifikationsprodukt, wenn die erste Zielnukleinsäure und/oder die zweite Zielnukleinsäure der einen oder der mehreren Malaria-Parasitenarten in der Probe vorhanden ist; und
(c) Nachweisen des Vorhandenseins oder Nichtvorhandenseins des Amplifikationsprodukts, wobei das Vorhandensein des Amplifikationsprodukts das Vorhandensein der einen oder der mehreren Malaria-Parasitenarten in der Probe anzeigt und wobei das Nichtvorhandensein des Amplifikationsprodukts das Nichtvorhandensein der einen oder der mehreren Malaria-Parasitenarten in der Probe anzeigt; und
wobei der eine oder die mehreren Sätze von Oligonukleotidprimern und die eine oder die mehreren Oligonukleotidsonden Folgendes umfassen:
(1) einen Satz von Oligonukleotidprimern und eine Oligonukleotidsonde für die erste Zielnukleinsäure der einen oder der mehreren Malaria-Parasitenarten, wobei der Satz von Oligonukleotidprimern Oligonukleotidprimer umfasst, umfassend die Nukleinsäuresequenzen von SEQ ID NO: 1 und 2; und wobei die Oligonukleotidsonde die Nukleinsäuresequenz von SEQ ID NO: 3 oder ein Komplement davon umfasst; und
(2) einen Satz von Oligonukleotidprimern und eine Oligonukleotidsonde für die zweite Zielnukleinsäure der einen oder der mehreren Malaria-Parasitenarten, wobei der Satz von Oligonukleotidprimern Oligonukleotidprimer umfasst, umfassend die Nukleinsäuresequenzen von SEQ ID NO: 13 und 14; und wobei die Oligonukleotidsonde die Nukleinsäuresequenz von SEQ ID NO: 15 oder ein Komplement davon umfasst.

3. Verfahren zum Nachweis einer oder mehrerer Malaria-Parasitenarten, die zur Gattung *Plasmodium* gehören, in einer Probe, wobei das Verfahren Folgendes umfasst:
(a) Durchführen eines Amplifikationsschritts, umfassend das Inkontaktbringen der Probe mit einem oder mehreren Sätzen von Oligonukleotidprimern, um ein Amplifikationsprodukt zu erzeugen, wenn eine Zielnukleinsäure der einen oder der mehreren Malaria-Parasitenarten in der Probe vorhanden ist;
(b) Durchführen eines Hybridisierungsschritts, umfassend das Inkontaktbringen einer oder mehrerer Oligonukleotidsonden mit dem Amplifikationsprodukt, wenn die Zielnukleinsäure der einen oder der mehreren Malaria-Parasitenarten in der Probe vorhanden ist; und
(c) Nachweisen des Vorhandenseins oder Nichtvorhandenseins des Amplifikationsprodukts, wobei das Vorhandensein des Amplifikationsprodukts das Vorhandensein der einen oder der mehreren Malaria-Parasitenarten in der Probe anzeigt und wobei das Nichtvorhandensein des Amplifikationsprodukts das Nichtvorhandensein der einen oder der mehreren Malaria-Parasitenarten in der Probe anzeigt; und
wobei der eine oder die mehreren Sätze von Oligonukleotidprimern und die eine oder die mehreren Oligonukleotidsonden einen Satz von Oligonukleotidprimern, umfassend Oligonukleotidprimer, umfassend die Nukleinsäuresequenzen von SEQ ID NO: 1 und 2; und eine Oligonukleotidsonde, umfassend die Nukleinsäuresequenz von SEQ ID NO: 3 oder ein Komplement davon, umfassen.

4. Verfahren zum Nachweis einer oder mehrerer Malaria-Parasitenarten, die zur Gattung *Plasmodium* gehören, in einer Probe, wobei das Verfahren Folgendes umfasst:
(a) Durchführen eines Amplifikationsschritts, umfassend das Inkontaktbringen der Probe mit einem oder mehreren Sätzen von Oligonukleotidprimern, um ein Amplifikationsprodukt zu erzeugen, wenn eine Zielnukleinsäure der einen oder der mehreren Malaria-Parasitenarten in der Probe vorhanden ist;
(b) Durchführen eines Hybridisierungsschritts, umfassend das Inkontaktbringen einer oder mehrerer Oligonukleotidsonden mit dem Amplifikationsprodukt, wenn die Zielnukleinsäure der einen oder der mehreren Malaria-Parasitenarten in der Probe vorhanden ist; und
(c) Nachweisen des Vorhandenseins oder Nichtvorhandenseins des Amplifikationsprodukts, wobei das Vorhandensein des Amplifikationsprodukts das Vorhandensein der einen oder der mehreren Malaria-Parasitenarten in der Probe anzeigt und wobei das Nichtvorhandensein des Amplifikationsprodukts das Nichtvorhandensein der einen oder der mehreren Malaria-Parasitenarten in der Probe anzeigt; und
wobei der eine oder die mehreren Sätze von Oligonukleotidprimern und die eine oder die mehreren Oligonukleotidsonden einen Satz von Oligonukleotidprimern, umfassend Oligonukleotidprimer, umfassend die Nukleinsäuresequenzen von SEQ ID NO: 13 und 14 oder Komplemente davon; und eine Oligonukleotidsonde, umfassend die Nukleinsäuresequenz von SEQ ID NO: 15 oder ein Komplement davon, umfassen.

5. Verfahren nach einem der Ansprüche 1 bis 4, wobei die eine oder die mehreren Malaria-Parasitenarten, die zur Gattung *Plasmodium* gehören, *P. falciparum*, *P. vivax*, *P. ovale*, *P. malariae* und/oder *P. knowlesi* sind.

6. Verfahren nach einem der Ansprüche 1 bis 5, wobei die Probe eine biologische Probe ist und ausgewählt ist aus Vollblut, Atemwegsproben, Urin, Stuhlproben, Blutproben, Plasma, Hautabstrichen, Nasenabstrichen, Wundabstrichen, Blutkulturen, Haut oder Weichteilinfektionen.

7. Verfahren nach einem der Ansprüche 1 bis 6, wobei mindestens eine der einen oder der mehreren Oligonukleotidsonden mit einer fluoreszierenden Donorgruppierung und einer entsprechenden Akzeptorgruppierung markiert ist.

8. Verfahren nach Anspruch 7, wobei Schritt (c) ferner das Nachweisen des Vorhandenseins oder Nichtvorhandenseins von Fluoreszenzresonanzenergietransfer (FRET) zwischen der fluoreszierenden Donorgruppierung und der Akzeptorgruppierung der einen oder der mehreren Oligonukleotidsonden umfasst, wobei das Vorhandensein oder Nichtvorhandensein von Fluoreszenz das Vorhandensein oder Nichtvorhandensein der einen oder der mehreren Malaria-Parasitenarten in der Probe anzeigt.

9. Kit zum Nachweis einer oder mehrerer Malaria-Parasitenarten, die zur Gattung *Plasmodium* gehören und in einer Probe vorhanden sein können, wobei das Kit Amplifikationsreagenzien, umfassend mindestens eines von einer DNA-Polymerase und Nukleotidmonomeren, und einen oder mehrere Sätze von Oligonukleotidprimern und eine oder mehrere Oligonukleotidsonden umfasst, wobei der eine oder die mehreren Sätze von Oligonukleotidprimern und die eine oder die mehreren Oligonukleotidsonden Folgendes umfassen:
(1) einen Satz von Oligonukleotidprimern, umfassend Oligonukleotidprimer, umfassend die Nukleinsäuresequenzen von SEQ ID NO: 1 und 2; und eine Oligonukleotidsonde, umfassend die Nukleinsäuresequenz von SEQ ID NO: 3 oder ein Komplement davon; und/oder
(2) einen Satz von Oligonukleotidprimern, umfassend Oligonukleotidprimer, umfassend die Nukleinsäuresequenzen von SEQ ID NO: 13 und 14; und eine Oligonukleotidsonde, umfassend die Nukleinsäuresequenz von SEQ ID NO: 15 oder ein Komplement davon.

10. Kit zum Nachweis einer ersten Zielnukleinsäure und/oder einer zweiten Zielnukleinsäure einer oder mehrerer Malaria-Parasitenarten, die zur Gattung *Plasmodium* gehören und in einer Probe vorhanden sein können, wobei das Kit Amplifikationsreagenzien, umfassend mindestens eines von einer DNA-Polymerase und Nukleotidmonomeren, und einen oder mehrere Sätze von Oligonukleotidprimern und eine oder mehrere Oligonukleotidsonden umfasst, wobei der eine oder die mehreren Sätze von Oligonukleotidprimern und die eine oder die mehreren Oligonukleotidsonden Folgendes umfassen:
(1) einen Satz von Oligonukleotidprimern und eine Oligonukleotidsonde für die erste Zielnukleinsäure der einen oder der mehreren Malaria-Parasitenarten, wobei der Satz von Oligonukleotidprimern Oligonukleotidprimer umfasst, umfassend die Nukleinsäuresequenzen von SEQ ID NO: 1 und 2; und wobei die Oligonukleotidsonde die Nukleinsäuresequenz von SEQ ID NO: 3 oder ein Komplement davon umfasst; und
(2) einen Satz von Oligonukleotidprimern und eine Oligonukleotidsonde für die zweite Zielnukleinsäure der einen oder der mehreren Malaria-Parasitenarten, wobei der Satz von Oligonukleotidprimern Oligonukleotidprimer umfasst, umfassend die Nukleinsäuresequenzen von SEQ ID NO: 13 und 14; und wobei die Oligonukleotidsonde die Nukleinsäuresequenz von SEQ ID NO: 15 oder ein Komplement davon umfasst.

11. Kit zum Nachweis einer oder mehrerer Malaria-Parasitenarten, die zur Gattung *Plasmodium* gehören und in einer Probe vorhanden sein können, wobei das Kit Amplifikationsreagenzien, umfassend mindestens eines von einer DNA-Polymerase und Nukleotidmonomeren, und einen oder mehrere Sätze von Oligonukleotidprimern und eine oder mehrere Oligonukleotidsonden umfasst,
wobei der eine oder die mehreren Sätze von Oligonukleotidprimern und die eine oder die mehreren Sonden Folgendes umfassen: einen Satz von Oligonukleotidprimern, umfassend Oligonukleotidprimer, umfassend die Nukleinsäuresequenzen von SEQ ID NO: 1 und 2; und eine Oligonukleotidsonde, umfassend die Nukleinsäuresequenz von SEQ ID NO: 3 oder ein Komplement davon.

12. Kit zum Nachweis einer oder mehrerer Malaria-Parasitenarten, die zur Gattung *Plasmodium* gehören und in einer Probe vorhanden sein können, wobei das Kit Amplifikationsreagenzien, umfassend mindestens eines von einer DNA-Polymerase und Nukleotidmonomeren, und einen oder mehrere Sätze von Oligonukleotidprimern und eine oder mehrere Oligonukleotidsonden umfasst,
wobei der eine oder die mehreren Sätze von Oligonukleotidprimern und die eine oder die mehreren Oligonukleotidsonden Folgendes umfassen: einen Satz von Oligonukleotidprimern, umfassend Oligonukleotidprimer, umfassend die Nukleinsäuresequenzen von SEQ ID NO: 13 und 14; und eine Oligonukleotidsonde, umfassend die Nukleinsäuresequenz von SEQ ID NO: 15 oder ein Komplement davon.

13. Kit nach einem der Ansprüche 9 bis 12, wobei die eine oder die mehreren Malaria-Parasitenarten, die zur Gattung *Plasmodium* gehören, *P. falciparum, P. vivax, P. ovale, P. malariae* und/oder *P. knowlesi* sind.

14. Kit nach einem der Ansprüche 9 bis 13, wobei die Probe eine biologische Probe ist und ausgewählt ist aus Vollblut, Atemwegsproben, Urin, Stuhlproben, Blutproben, Plasma, Hautabstrichen, Nasenabstrichen, Wundabstrichen, Blutkulturen, Haut oder Weichteilinfektionen.

15. Kit nach einem der Ansprüche 9 bis 14, wobei mindestens eine der einen oder der mehreren Oligonukleotidsonden mit einer fluoreszierenden Donorgruppierung und einer entsprechenden Akzeptorgruppierung markiert ist.

## Revendications

1. Procédé pour la détection d'une ou de plusieurs espèces de parasites du paludisme appartenant au genre *Plasmodium* dans un échantillon, le procédé comprenant :
(a) la réalisation d'une étape d'amplification comprenant la mise en contact de l'échantillon avec un ou plusieurs ensembles d'amorces oligonucléotidiques pour produire un produit d'amplification, si un acide nucléique cible de la ou des espèces de parasites du paludisme est présent dans l'échantillon ;
(b) la réalisation d'une étape d'hybridation comprenant la mise en contact d'une ou plusieurs sondes oligonucléotidiques avec le produit d'amplification, si un acide nucléique cible de la ou des espèces de parasites du paludisme est présent dans l'échantillon ; et
(c) la détection de la présence ou de l'absence du produit d'amplification, la présence du produit d'amplification étant un indicateur de la présence de la ou des espèces de parasites du paludisme dans l'échantillon, et l'absence du produit d'amplification étant un indicateur de l'absence de la ou des espèces de parasites du paludisme dans l'échantillon ; et
dans lequel le ou les ensembles d'amorces oligonucléotidiques et la ou les sondes oligonucléotidiques comprennent :
(1) un ensemble d'amorces oligonucléotidiques comprenant des amorces oligonucléotidiques comprenant les séquences d'acide nucléique de SEQ ID NO : 1 et 2 ; et une sonde oligonucléotidique comprenant la séquence d'acide nucléique de SEQ ID NO : 3, ou un complément de celle-ci ; et/ou
(2) un ensemble d'amorces oligonucléotidiques comprenant des amorces oligonucléotidiques comprenant les séquences d'acide nucléique de SEQ ID NO : 13 et 14 ; et une sonde oligonucléotidique comprenant la séquence d'acide nucléique de SEQ ID NO : 15, ou un complément de celle-ci.

2. Procédé pour la détection d'un premier acide nucléique cible et/ou d'un second acide nucléique cible d'une ou de plusieurs espèces de parasites du paludisme appartenant au genre *Plasmodium* dans un échantillon, le procédé comprenant :
(a) la réalisation d'une étape d'amplification comprenant la mise en contact de l'échantillon avec un ou plusieurs ensembles d'amorces oligonucléotidiques pour produire un produit d'amplification, si le premier acide nucléique cible et/ou le second acide nucléique cible de la ou des espèces de parasites du paludisme est présent dans l'échantillon ;
(b) la réalisation d'une étape d'hybridation comprenant la mise en contact d'une ou de plusieurs sondes oligonucléotidiques avec le produit d'amplification, si le premier acide nucléique cible et/ou le second acide nucléique cible de la ou des espèces de parasites du paludisme est présent dans l'échantillon ; et
(c) la détection de la présence ou de l'absence du produit d'amplification, la présence du produit d'amplification étant un indicateur de la présence de la ou des espèces de parasites du paludisme dans l'échantillon, et l'absence du produit d'amplification étant un indicateur de l'absence de la ou des espèces de parasites du paludisme dans l'échantillon ; et
dans lequel le ou les ensembles d'amorces oligonucléotidiques et la ou les sondes oligonucléotidiques comprennent :
(1) un ensemble d'amorces oligonucléotidiques et une sonde oligonucléotidique pour le premier acide nucléique cible de la ou des espèces de parasites du paludisme, l'ensemble d'amorces oligonucléotidiques comprenant des amorces oligonucléotidiques comprenant les séquences d'acide nucléique de SEQ ID NO : 1 et 2 ; et la sonde oligonucléotidique comprenant la séquence d'acide nucléique de SEQ ID NO : 3, ou un complément de celle-ci ; et
(2) un ensemble d'amorces oligonucléotidiques et une sonde oligonucléotidique pour le second acide nucléique cible de la ou des espèces de parasites du paludisme, l'ensemble d'amorces oligonucléotidiques comprenant des amorces oligonucléotidiques comprenant les séquences d'acide nucléique de SEQ ID NO : 13 et 14 ; et la sonde oligonucléotidique comprenant la séquence d'acide nucléique de SEQ ID NO : 15, ou un complément de celle-ci.

3. Procédé pour la détection d'une ou de plusieurs espèces de parasites du paludisme appartenant au genre *Plasmodium* dans un échantillon, le procédé comprenant :
(a) la réalisation d'une étape d'amplification comprenant la mise en contact de l'échantillon avec un ou plusieurs ensembles d'amorces oligonucléotidiques pour produire un produit d'amplification, si un acide nucléique cible de la ou des espèces de parasites du paludisme est présent dans l'échantillon ;
(b) la réalisation d'une étape d'hybridation comprenant la mise en contact d'une ou de plusieurs sondes oligonucléotidiques avec le produit d'amplification, si l'acide nucléique cible de la ou des espèces de parasites du paludisme est présent dans l'échantillon ; et
(c) la détection de la présence ou de l'absence du produit d'amplification, la présence du produit d'amplification étant un indicateur de la présence de la ou des espèces de parasites du paludisme dans l'échantillon, et l'absence du produit d'amplification étant un indicateur de l'absence de la ou des espèces de parasites du paludisme dans l'échantillon ; et
dans lequel le ou les ensembles d'amorces oligonucléotidiques et la ou les sondes oligonucléotidiques comprennent un ensemble d'amorces oligonucléotidiques comprenant des amorces oligonucléotidiques comprenant les séquences d'acide nucléique de SEQ ID NO : 1 et 2 ; et une sonde oligonucléotidique comprenant la séquence d'acide nucléique de SEQ ID NO : 3, ou un complément de celle-ci.

4. Procédé pour la détection d'une ou de plusieurs espèces de parasites du paludisme appartenant au genre *Plasmodium* dans un échantillon, le procédé comprenant :
(a) la réalisation d'une étape d'amplification comprenant la mise en contact de l'échantillon avec un ou plusieurs ensembles d'amorces oligonucléotidiques pour produire un produit d'amplification, si un acide nucléique cible de la ou des espèces de parasites du paludisme est présent dans l'échantillon ;
(b) la réalisation d'une étape d'hybridation comprenant la mise en contact d'une ou de plusieurs sondes oligonucléotidiques avec le produit d'amplification, si l'acide nucléique cible de la ou des espèces de parasites du paludisme est présent dans l'échantillon ; et
(c) la détection de la présence ou de l'absence du produit d'amplification, la présence du produit d'amplification étant un indicateur de la présence de la ou des espèces de parasites du paludisme dans l'échantillon, et l'absence du produit d'amplification étant un indicateur de l'absence de la ou des espèces de parasites du paludisme dans l'échantillon ; et
dans lequel le ou les ensembles d'amorces oligonucléotidiques et la ou les sondes oligonucléotidiques comprennent un ensemble d'amorces oligonucléotidiques comprenant des amorces oligonucléotidiques comprenant les séquences d'acide nucléique de SEQ ID NO : 13 et 14, ou des compléments de celles-ci ; et une sonde oligonucléotidique comprenant la séquence d'acide nucléique de SEQ ID NO : 15, ou un complément de celle-ci.

5. Procédé selon l'une quelconque des revendications 1 à 4, dans lequel la ou les espèces de parasites du paludisme qui appartiennent au genre *Plasmodium* sont *P. falciparum, P. vivax, P. ovale, P. malariae,* et/ou *P. knowlesi.*

6. Procédé selon l'une quelconque des revendications 1 à 5, dans lequel l'échantillon est un échantillon biologique et est choisi parmi du sang total, des échantillons respiratoires, de l'urine, des échantillons fécaux, des échantillons sanguins, du plasma, des écouvillons dermiques, des écouvillons nasaux, des écouvillons de plaies, des hémocultures, de la peau ou des infections de tissus mous.

7. Procédé selon l'une quelconque des revendications 1 à 6, dans lequel au moins une parmi la ou les sondes oligonucléotidiques est marquée par une fraction fluorescente donneuse et une fraction acceptrice correspondante.

8. Procédé selon la revendication 7, dans lequel l'étape (c) comprend en outre la détection de la présence ou de l'absence d'un transfert d'énergie par résonance de fluorescence (FRET) entre la fraction fluorescente donneuse et la fraction acceptrice de la ou des sondes oligonucléotidiques, dans lequel la présence ou l'absence de fluorescence indique la présence ou l'absence de la ou des espèces de parasites du paludisme dans l'échantillon.

9. Kit pour la détection d'une ou de plusieurs espèces de parasites du paludisme appartenant au genre *Plasmodium* qui peuvent être présentes dans un échantillon, le kit comprenant des réactifs d'amplification comprenant au moins un parmi une ADN polymérase et des monomères nucléotidiques, et un ou plusieurs ensembles d'amorces oligonucléotidiques et une ou plusieurs sondes oligonucléotidiques, dans lequel le ou les ensembles d'amorces oligonucléotidiques et la ou les sondes oligonucléotidiques comprennent :
(1) un ensemble d'amorces oligonucléotidiques comprenant des amorces oligonucléotidiques comprenant les séquences d'acide nucléique de SEQ ID NO : 1 et 2 ; et une sonde oligonucléotidique comprenant la séquence d'acide nucléique de SEQ ID NO : 3, ou un complément de celle-ci ; et/ou
(2) un ensemble d'amorces oligonucléotidiques comprenant des amorces oligonucléotidiques comprenant les séquences d'acide nucléique de SEQ ID NO : 13 et 14 ; et une sonde oligonucléotidique comprenant la séquence d'acide nucléique de SEQ ID NO : 15, ou un complément de celle-ci.

10. Kit pour la détection d'un premier acide nucléique cible et/ou d'un second acide nucléique cible d'une ou de plusieurs espèces de parasites du paludisme appartenant au genre *Plasmodium* qui peuvent être présentes dans un échantillon, le kit comprenant des réactifs d'amplification comprenant au moins un parmi une ADN polymérase et des monomères nucléotidiques, et un ou plusieurs ensembles d'amorces oligonucléotidiques et une ou plusieurs sondes oligonucléotidiques, dans lequel le ou les ensembles d'amorces oligonucléotidiques et la ou les sondes oligonucléotidiques comprennent :
(1) un ensemble d'amorces oligonucléotidiques et une sonde oligonucléotidique pour le premier acide nucléique cible de la ou des espèces de parasites du paludisme, l'ensemble d'amorces oligonucléotidiques comprenant des amorces oligonucléotidiques comprenant les séquences d'acide nucléique de SEQ ID NO : 1 et 2 ; et la sonde oligonucléotidique comprenant la séquence d'acide nucléique de SEQ ID NO : 3, ou un complément de celle-ci ; et
(2) un ensemble d'amorces oligonucléotidiques et une sonde oligonucléotidique pour le second acide nucléique cible de la ou des espèces de parasites du paludisme, l'ensemble d'amorces oligonucléotidiques comprenant des amorces oligonucléotidiques comprenant les séquences d'acide nucléique de SEQ ID NO : 13 et 14 ; et la sonde oligonucléotidique comprenant la séquence d'acide nucléique de SEQ ID NO : 15, ou un complément de celle-ci.

11. Kit pour la détection d'une ou de plusieurs espèces de parasites du paludisme appartenant au genre *Plasmodium* qui peuvent être présentes dans un échantillon, le kit comprenant des réactifs d'amplification comprenant au moins un parmi une ADN polymérase et des monomères nucléotidiques, et un ou plusieurs ensembles d'amorces oligonucléotidiques et une ou de plusieurs sondes oligonucléotidiques,
dans lequel le ou les ensembles d'amorces oligonucléotidiques et la ou les sondes comprennent : un ensemble d'amorces oligonucléotidiques comprenant des amorces oligonucléotidiques comprenant les séquences d'acide nucléique de SEQ ID NO : 1 et 2 ; et une sonde oligonucléotidique comprenant la séquence d'acide nucléique de SEQ ID NO : 3, ou un complément de celle-ci.

12. Kit pour la détection d'une ou de plusieurs espèces de parasites du paludisme appartenant au genre *Plasmodium* qui peuvent être présentes dans un échantillon, le kit comprenant des réactifs d'amplification comprenant au moins un parmi une ADN polymérase et des monomères nucléotidiques, et un ou plusieurs ensembles d'amorces oligonucléotidiques et une ou de plusieurs sondes oligonucléotidiques,
dans lequel le ou les ensembles d'amorces oligonucléotidiques et la ou les sondes oligonucléotidiques comprennent : un ensemble d'amorces oligonucléotidiques comprenant des amorces oligonucléotidiques comprenant les séquences d'acide nucléique de SEQ ID NO : 13 et 14 ; et une sonde oligonucléotidique comprenant la séquence d'acide nucléique de SEQ ID NO : 15, ou un complément de celle-ci.

13. Kit selon l'une quelconque des revendications 9 à 12, dans lequel la ou les espèces de parasites du paludisme qui appartiennent au genre *Plasmodium* sont *P. falciparum, P. vivax, P. ovale, P. malariae,* et/ou *P. knowlesi.*

14. Kit selon l'une quelconque des revendications 9 à 13, dans lequel l'échantillon est un échantillon biologique et est choisi parmi du sang total, des échantillons respiratoires, de l'urine, des échantillons fécaux, des échantillons sanguins, du plasma, des écouvillons dermiques, des écouvillons nasaux, des écouvillons de plaies, des hémocultures, de la peau ou des infections de tissus mous.

15. Kit selon l'une quelconque des revendications 9 à 14, dans lequel au moins une parmi la ou les sondes oligonucléotidiques est marquée par une fraction fluorescente donneuse et une fraction acceptrice correspondante.
